# EUROPEAN PATENT SPECIFICATION

(11) **EP 2 729 002 B1**
(45) Date of publication and mention of the grant of the patent: **17.08.2016**
(21) Application number: 12745648.1
(22) Date of filing: 03.07.2012
(51) Int. Cl.: A01N 25/28, A01N 43/12, A01N 43/90, A01N 51/00

(54) **MICELLE-COATED ORGANIC CRYSTALLINE PARTICLES**
MIZELL-BESCHICHTETE ORGANISCHE KRISTALLINE PARTIKEL
PARTICULES CRISTALLINES ORGANIQUES ENDUITS AVEC DES MICELLES

(30) Priority: 04.07.2011 GB 201111438
(43) Date of publication of application: 14.05.2014
(73) Proprietor: Syngenta Limited, Guildford, Surrey GU2 7YH (GB)
(72) Inventor: MULQUEEN, Patrick, Joseph, Bracknell Berkshire RG42 6EY (GB); THOMSON, Niall, Rae, Bracknell Berkshire RG42 6EY (GB); BIGGS, Simon, Richard, Leeds LS2 9DF (GB); CHAGNEUX, Nelly, Leeds LS2 9DF (GB); DUBOIS, Mathieu, Edmond, René, Leeds LS2 9DF (GB); SARKER, Prodip, Leeds LS2 9DF (GB); SCANLON, Shane, Leeds LS2 9DF (GB)
(74) Representative: Syngenta International AG
(86) International application number: PCT/EP2012/062946
(87) International publication number: WO 2013/004705

(56) References cited:
- WO-A1-00/77281
- WO-A1-96/00567
- US-A1- 2008 057 128
- US-A1- 2010 323 884
- TIMOTHY ADDISON ET AL: "Polymeric Microcapsules Assembled from a Cationic/Zwitterionic Pair of Responsive Block Copolymer Micelles", LANGMUIR, vol. 26, no. 9, 4 May 2010 (2010-05-04), pages 6281-6286, XP55040897, ISSN: 0743-7463, DOI: 10.1021/la904064d
- PHUONG M. NGUYEN ET AL: "Extended Release Antibacterial Layer-by-Layer Films Incorporating Linear-Dendritic Block Copolymer Micelles", CHEMISTRY OF MATERIALS, vol. 19, no. 23, 1 November 2007 (2007-11-01), pages 5524-5530, XP55040892, ISSN: 0897-4756, DOI: 10.1021/cm070981f
- YAN G W ET AL: "Aggregation of hollow CaCO3 spheres by calcite nanoflakes", MATERIALS RESEARCH BULLETIN, ELSEVIER, KIDLINGTON, GB, vol. 43, no. 8-9, 4 August 2008 (2008-08-04), pages 2069-2077, XP022717546, ISSN: 0025-5408, DOI: 10.1016/J.MATERRESBULL.2007.09.014 [retrieved on 2008-06-12]
- GAUCHER G ET AL: "Block copolymer micelles: preparation, characterization and application in drug delivery", JOURNAL OF CONTROLLED RELEASE, ELSEVIER, AMSTERDAM, NL, vol. 109, no. 1-3, 5 December 2005 (2005-12-05), pages 169-188, XP027664302, ISSN: 0168-3659 [retrieved on 2005-12-05]
- F. M. MENGER ET AL: "Cross-Linked Micelles", LANGMUIR, vol. 11, no. 6, 1 June 1995 (1995-06-01), pages 1855-1857, XP55041171, ISSN: 0743-7463, DOI: 10.1021/la00006a005

## Description

The present invention relates to organic crystalline particles coated with micelles of AB block copolymers, to compositions comprising such particles, to a process for preparing the coated particles and to uses of the particles and the compositions [for example to produce surface coatings with high loadings of copolymer and uses of products derived therefrom].

Effective coating of small particles such as organic crystals with a polymer is difficult to achieve. Many techniques have been applied to coat particles, such as those based on Wurster coating technologies for spray dry coatings wherein a fluidised bed of dry particles has a coating solution sprayed onto the fluidised bed and solidified on the particles by either evaporation of a volatile solvent in the coating solution or cooling to set the coating polymer (if applied in molten form). Such techniques are notoriously variable, in that it is difficult to avoid agglomeration of the particles into larger masses and the coating can be extremely ineffective in coating all surfaces and edges of a particle (especially a small crystalline particle with variable edges and sides of crystal). This limitation can be partially overcome by employing higher quantities of coating composition but this can significantly alter the properties of the organic particle being coated as well as having an impact on the economics of the process and product cost.

Coating of particles in a liquid medium is highly attractive if a technique could be identified. Work with dispersions of organic pesticides in water (WO2006/015791) in the presence of reactive monomers produced "coated" particles as dispersions in water but these are matrix particles where the particle is engulfed in a polymer during the polymerisation process. Many similar techniques produce such matrix particles.

Surfactants are recognised to adsorb uniquely to interfaces such as oil/water interfaces and solid/liquid interfaces and are employed as stabilisers to produce dispersions of particles in a liquid medium (such as water) that remain stable to agglomeration on storage. Because of this property of adsorbing as a monomolecular layer at an interface, surfactants in the form of polyelectrolytes have been employed to produce layers of surfactant on a substrate such as a solid particle. Such processes (for example, as in WO2000/077281) are slow to build up oppositely charged single layers of polyelectrolyte (each layer being only the thickness of a surfactant monolayer and many layers being required to build up a coating thickness of utility). Surfactants can also aggregate into structures containing many surfactant molecules in a single aggregate. These aggregates are called micelles. They are commonly spherical in shape but can have a large range of shapes and structures. The number of molecules that compose such an aggregate can be very many, often in the order of hundreds of molecules. Micelles can be composed of relatively simple surfactant structures but can also be composed of high molecular weight block-copolymer surfactants. Moreover, even large complex block copolymers can form micelles. Such block copolymer micelles, when comprised of oppositely charged micelles, have been induced to adsorb in a layer by layer (LbL) manner onto spherical colloidal particles to produce coatings on particles such as a latex or a spherical silica particle (NSTI-Nanotech 2007, www.nsti.org, ISBN 1420061836 Vol. 2, 2007 pp13-16 and Adv. Mater. 2007, 19, 247-250).

Timothy Addison et al., Langmuir, vol. 26, no. 9, 4 May 2010, pages 6281-6286 discloses the use of spherical calcium carbonate particles. US2010/323884A1 discloses a loaded inverse micelle which may be applied to metal oxide nanoparticles. Phuong M. Nguyen et al., Chemistry of Materials, vol.19, no. 23, 1 November 2007, pages 5524-5530 discloses the preparation of micelles from polypropyleneoxide-poly(amidoamine) which are applied layer-by-layer to quartz. WO96/00567A1 discloses the use of block copolymer surfactants as stabilizer coatings for nanocrystal formulations.

We have now unexpectedly and surprisingly found that the use of complex copolymer micelles as coating agents for crystalline particles produces surface coatings with high loadings of copolymer in a single treatment (or very few treatments) and such products find utility in a range of applications, particularly the agrochemical field.

In one aspect, the present invention provides an organic crystalline particle according to claim 1.

WO08071957 and WO10038046 describe the chemistry of AB block copolymers that can form micelle structures and can be employed to surface coat structures such as fabrics, concrete structures, glass windscreens, glass structures to render them "stay-clean" by a combination of dust- repellence and water-sheeting effects. Such structures are large (in colloid terms). AB block copolymers mentioned in WO08071957 and WO10038046 are suitable for use in the present invention but other AB block copolymers are also of relevance for the present invention.

A coated crystalline particle according to the present invention may be prepared from a coating system derived from:
(a) an AB block copolymer; and
(b) a liquid medium;
where the AB block copolymer comprises:
(i) a first hydrophobic block A, comprising a polymer selected from the group consisting of a homopolymer of an acrylate or alkylacrylate monomer; a copolymer comprising two or three different monomers selected from acrylate or alkylacrylate monomers; a homopolymer of a styrenic derivative monomer; a copolymer comprising two different monomers selected from styrenic derivative monomers; a homopolymer of an alkene or diene monomer; a copolymer comprising two different monomers selected from alkene and diene monomers; a homopolymer of a heterocyclic monomer; and a random, alternating, gradient or block copolymer comprising monomers selected from acrylate monomers, alkylacrylate monomers, styrenic derivative monomers, alkene monomers and diene monomers; and
(ii) either a second hydrophobic block B or a hydrophilic block B having a different affinity than the block A for the liquid medium in which the AB copolymers are dispersed such that micelles are formed.

The key difference between block A and block B is that the two blocks have different affinities for, or solubilities in, the liquid; indeed block A and block B may even belong to the same chemical type, provided that they are sufficiently chemically different [for example, by virtue of different substitution patterns] such that they have different affinities for, or solubilities in, the liquid.

The liquid medium comprises either :
(i) water; or
(ii) an organic solvent or mixture of organic solvents; or
(iii) an organic solvent free from [or substantially free from] water; or
(iv) an organic solvent and water.

The term "organic solvent" means an organic polar or apolar solvent (for example, an oil). The liquid medium further optionally comprises one or more additives (selected from, for example, pH modifiers, surfactants and wetting agents).

Therefore, the present invention relies upon an AB block copolymer comprising two blocks (A and B) which have different affinities for a liquid medium such that micelles form in the liquid medium.

Although the micelles are formed in a liquid medium, any eventual coated particles may be present not only in not only a liquid composition but alternatively in a dry, solid composition [for instance, due to an evaporation step or a drying step]; in one aspect the present invention provides a composition comprising a plurality of coated crystalline particles as described herein, where in one aspect the composition is a solid composition and in an alternative aspect it comprises particles dispersed in a liquid [where the liquid may comprise water or may be non-aqueous].

Disclosed is an AB block copolymer which comprises:
(i) a first hydrophobic block A, comprising a polymer selected from the group consisting of a homopolymer of an acrylate or alkylacrylate (preferably an acrylate or C₁₋₄ alkylacrylate; more preferably an acrylate or methacrylate) monomer; a copolymer comprising two or three different monomers selected from acrylate or alkylacrylate (preferably an acrylate or C₁₋₄ alkylacrylate; more preferably an acrylate or methacrylate) monomers; a homopolymer of a styrenic derivative monomer; a copolymer comprising two different monomers selected from styrenic derivative monomers; a homopolymer of an alkene or diene monomer; a copolymer comprising two different monomers selected from alkene and diene monomers; a homopolymer of a heterocyclic monomer; a copolymer comprising two different monomers selected from heterocyclic monomers; and a random, alternating, gradient or block copolymer comprising monomers selected from acrylate monomers, alkylacrylate (preferably C₁₋₄ alkylacrylate; more preferably methacrylate) monomers, styrenic derivative monomers, alkene monomers, diene monomers and heterocyclic monomers; and
(ii) either a second hydrophobic block B or a hydrophilic block B having a different affinity than the block A for the liquid medium in which the AB copolymers are dispersed such that micelles are formed.

Throughout the discussion of the present invention, references to alkyl and alkylene groups and moieties, relate to both straight-chained and branched versions.

Preferably any acrylate or alkylacrylate monomer is, independently, of formula A' 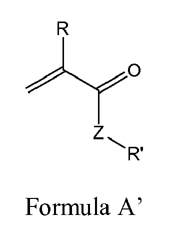 wherein R is H or a C₁ to C₄ alkyl chain; Z is O, a phosphorous derivative [preferably PH₃] or a nitrogen derivative [preferably NH]; R' is selected from the group comprising: C₁ to C₁₈ alkyl; alkylaminoalkylene containing from 1 to 18 carbon atoms (preferably from 2 to 18 carbon atoms); alkoxyalkylene containing from 1 to 18 carbon atoms (preferably from 2 to 18 carbon atoms); C₁ to C₁₈ dihydroxyalkyl; C₁ to C₁₈ silylalkyl; C₁ to C₁₈ epoxy alkyl; phosphoryl; phosphoryl C₁ to C₁₈ alkyl; a vinyl phosphonate or phosphoric acid monomer; and a methacrylate having at least one crosslinkable function or one UV or thermal-responsive unit; where each alkyl or alkylene group is, independently, fluorinated or non-fluorinated.

Preferably any styrenic derivative monomer is, independently, of formula B' 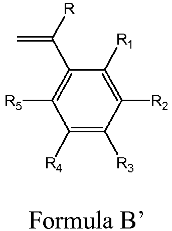 wherein R is H or a C₁ to C₄ alkyl group; and R₁, R₂, R₃, R₄ and R₅ are each independently H or a C₁ to C₈ alkyl group or a halogen atom [preferably chlorine or fluorine].

Preferably any alkene or diene monomer is, independently, of formula Cₐ or C_{b} 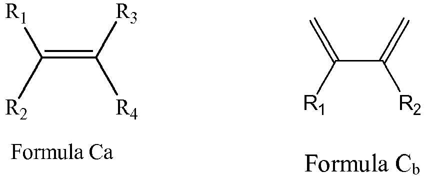 wherein R₁, R₂, R₃ and R₄ are each independently selected from H and C₁ to C₄ alkyl (preferably R₁, R₃ and R₄ are each H; and R₂ is H or C₁ to C₄ alkyl).

Preferably any heterocyclic monomer is, independently, of formula Dₐ, D_{b}, D_{c} or D_{d} 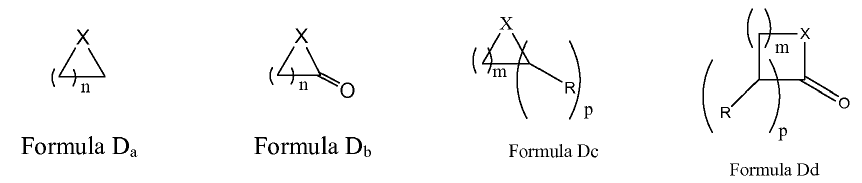 wherein n is from 1 to 7, m is from 0 to 5 and p is from 1 to 7; R is H or a C₁ to C8 alkyl group; and X is O, N or S.

The ratio of the monomers in each block of block copolymer AB is such that the weight fraction of the (hydrophobic) block A and the (hydrophobic or hydrophilic) block B agents leads to the formation of organised aggregates, such as micelles. The number of the monomers comprising the block copolymer AB is: preferably from 5 to 250 units of A; more preferably from 10 to 200 units of A; and most preferably from 15 to 150 units of A; and, likewise, preferably from 5 to 250 units of B; more preferably from 10 to 200 units of B; and most preferably from 15 to 150 units of B.

A suitable alkylacrylic or acrylate monomer of Formula A' is when Z is O; and R' is a C₁ to C₁₈ alkyl group (more preferably a C₁ to C₈ alkyl group); another suitable monomer of Formula A' is provided by Formula 1: 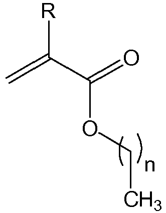 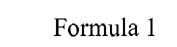 where n is 1 to 17, more preferably 1 to 8.

A suitable fluorinated alkylacrylic or acrylate monomer of Formula A' is when Z is O; and R' is a fluorinated alkyl group; another suitable monomer of Formula A' is provided by Formula 2: 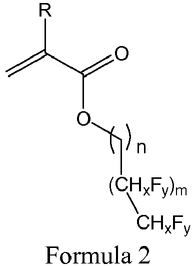 where n is 1 to 6 and the chain is linear or non-linear, more preferably 1 or 2; m is 0 to 7 and the chain is linear or non-linear, x is 0 to 2 and y is 3-x.

A suitable alkylacrylic or acrylate monomer for Formula A' is when Z is O; and R' is an alkylaminoalkyl group containing up to eighteen carbon atoms. Another suitable monomer of Formula A' is provided by Formula 3: 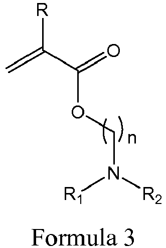 where R₁ and R₂ are each independently H, a C₁ to C₆ alkyl group; phenyl; benzyl or cyclohexyl; and n is from 1 to 17; more preferably, R₁ and R₂ are each methyl and n is from 1 to 5.

A suitable alkylacrylic or acrylate monomer for Formula A' is when Z is O; and R' is an hydroxyalkyl containing up to 18 carbon atoms. Another suitable monomer of Formula A' is provided by Formula 4a or 4b: 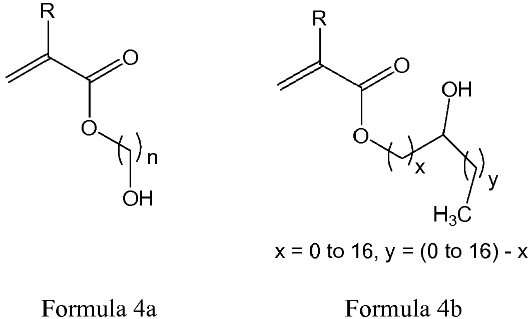 where n is 1 to 18 and the chain is linear or non-linear (more preferably n is from 1 to 4) and x and y are each 0 to 16, more preferably 0 to 6. Suitably, for Formula 4b, x = 0 to 16; y = 0 to 16; and x + y ≤ 16.

A suitable alkylacrylic or acrylate monomer for Formula A' is when Z is O; and R' comprises a dihydroxyalkyl group. Another suitable monomer of Formula A' is provided by Formula 5a or 5b: 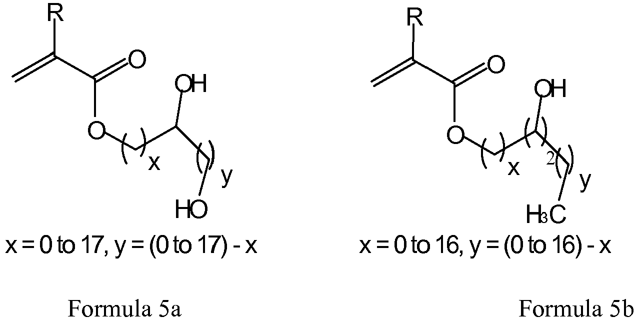 where x and y are each 0 to 17 in Formula 5a or 0 to 16 in Formula 5b; more preferably x and y are each 0 to 7 in Formula 5a or 0 to 6 in Formula 5b (and the chain can be linear or non-linear). Suitably, for Formula 5a, x = 0 to 17; y = 0 to 17; and x + y ≤ 17. Suitably, for Formula 5b, x = 0 to 16; y = 0 to 16; and x + y ≤ 16.

A suitable alkylacrylic or acrylate monomer for Formula A' is when Z is O; and R' is a C₁ to C₁₇ silylalkyl group. Another suitable monomer of Formula A' is provided by Formula 6a or 6b: 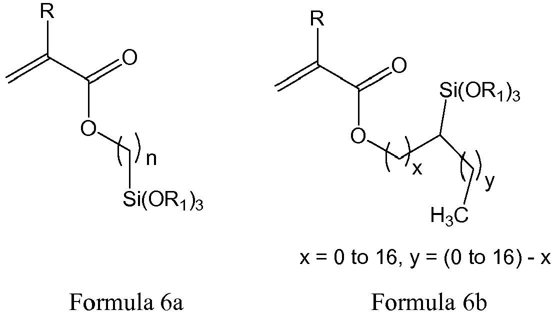 where R₁ is H or C₁ to C₄ alkyl and x and y are each from 0 to 16, preferably from 1 to 6. Suitably, for Formula 6b, x = 0 to 16; y = 0 to 16; and x + y ≤ 16.

A suitable alkylacrylic or acrylate monomer for Formula A' is when Z is O; and R' is an epoxy alkyl group. Another suitable monomer of Formula A' is provided by Formula 7a or 7b: 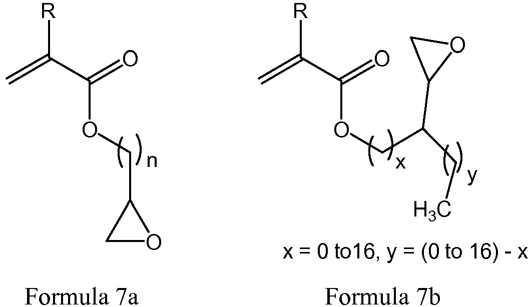 where x and y are each from 0 to 16, preferably from 0 to 6. Suitably, for Formula 7b, x = 0 to 16; y = 0 to 16; and x + y ≤ 16.

A suitable monomer of Formula A' is when Z is O; and R' is a phosphoryl or phosphoryl alkyl group. Another suitable monomer of Formula A' is provided by Formula 8a or 8b: 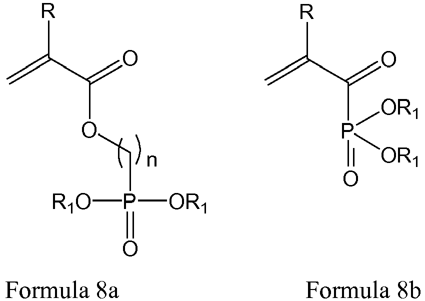 where each R₁ is independently H or C₁ to C₆ alkyl, preferably H or methyl.

Suitable monomers of Formula B' are independently selected from styrene, α-methylstyrene, 2-methylstyrene, 4-methylstyrene, 2,4-dimethylstyrene, 2,4,6-trimethylstyrene, 4-isopropylstyrene, 2-fluorostyrene, 3-fluorostyrene, 4-fluorostyrene, 2,6-difluorostyrene, 2,3,4,5,6-pentafluorostyrene, 2-chlorostyrene, 3-chlorostyrene, 4-chlorostyrene and 2,6-dichlorostyrene and other vinyl substituted aromatics.

Suitable monomers of Formula Cₐ or C_{b} are independently selected from ethylene, propylene, butylene, butadiene and isoprene.

Suitable monomers of Formula Dₐ or D_{b} or D_{c} or D_{d} are independently selected from ethylene oxide, propylene oxide, butylene oxide and caprolactone type monomers (such as ε-caprolactone or γ-butyrolactone, lactide, oxiran-2-one, 1,3-dioxolane and caprolactam).

When the Block B is hydrophobic, it may comprise one or more monomers, independently selected from the monomers defined above. Block B is chosen to have a different affinity to the liquid medium to Block A. The structures outlined for Block A can be applied for Block B provided Block A and B are different to each other.

When the block B is hydrophilic, a number of chemicals may be employed for the hydrophilic component B, all of which need to be water-soluble; examples may be selected from the group comprising:
hydrophilic organic monomers, oligomers, prepolymers or copolymers derived from vinyl alcohol, N-vinylpyrrolidone, N-vinyl lactam, acrylamide, amide, styrenesulfonic acid, combinations of vinylbutyral and N-vinylpyrrolidone, methacrylic acid, acrylic acid, vinylmethyl ether, vinylpyridylium halide, melamine, maleic anhydride/methyl vinyl ether, vinylpyridine, ethyleneoxide, ethyleneoxide ethylene imine, glycol, vinyl acetate, vinyl acetate/crotonic acid, methyl cellulose, ethyl cellulose, carboxymethyl cellulose, hydroxyethyl cellulose, hydroxypropyl cellulose, hydroxymethyl ethyl cellulose, hydroxypropylmethyl cellulose, cellulose acetate, cellulose nitrate, hydroxyalkyl (alkyl)acrylate such as hydroxyethyl (meth)acrylate, hydroxypropyl (meth)acrylate, alkylaminoalkyl (alkyl)acrylate, 2-(dimethyl amino) ethyl methacrylate, 2-(diethyl amino) ethyl methacrylate, 2-(diisopropyl amino) ethyl methacrylate, 2-(N-morpholino)ethyl methacrylate, or a derivative thereof, ethylene glycol (meth)acrylates (for example triethylene glycol (meth)acrylate) and (meth)acrylamide), N-alkyl (meth) acrylamides (for example N-methyl (meth)acrylamide and N-hexyl (meth)acrylamide), N,N-dialkyl (meth)acrylamides (for example N,N-dimethyl (meth)acrylamide and poly-N,N-dipropyl (meth)acrylamide), N-hydroxyalkyl (meth)acrylamide polymers, such as poly-N-methylol (meth)acrylamide and poly-N-hydroxy ethyl (meth)acrylamide, and N,N-dihydroxyalkyl (meth)acrylamide polymers, such as poly-N,N-dihydroxyethyl (meth)acrylamide, ether polyols, polyethylene oxide, polypropylene oxide, and poly(vinyl ether), alkylvinyl sulfones, alkylvinylsulfone-acrylates, (alkyl)acrylate with a pendent phosphorus group such as vinylphosphonate, vinylphosphonic acid, vinylphosphine oxide and any (alkyl)acrylate with a ester function -COOR such as R is CₓH₂ₓPO₃R₂ wherein x is 2 to 10, most preferably x is 2, and R is a hydrogen or an alkyl group having 1 to 4 carbon atoms, preferably methyl; and related compounds or a combination thereof.

In accordance with the present invention the polymers comprising the AB block copolymer comprise monomers, and the ratio of the monomers comprising each polymer of the block copolymer AB is such that the weight ratio of the (hydrophobic) block A to the (hydrophobic or hydrophilic) block B leads to the formation of organised aggregates. In addition, the weight fraction of the (hydrophobic) block A and the (hydrophobic or hydrophilic) block B leads to the formation of micelles. Certain copolymers used in the present invention have been found to form complex and large micelles in solution.

As stated above AB Block copolymers can comprise a hydrophobic ("water hating") block A and a second hydrophilic ("water loving") block B; or two hydrophobic blocks A and B may be differentiated by having different solubility parameters in the same liquid medium. Variation in the copolymer properties can be obtained by varying the monomer types (different available chemistries), the molecular weights of the copolymer (at a fixed ratio of the two component block sizes) and the ratio of the molecular weights of the constituent blocks (at a fixed overall molecular weight for the copolymer).

Importantly, to form micelles (that is, aggregates formed by molecules of block coplymer) in a liquid medium, the insoluble (or poorly soluble in the liquid medium) blocks drive the formation of aggregates of the molecules. The structures of the aggregates are dependent on the copolymer concentration and the exact nature of the copolymer molecules. Schematically, micelles may be seen, for example, as a spherical aggregate having two parts; one core composed of the copolymer block insoluble or less soluble in the liquid medium and one corona composed of a copolymer block having affinity for the liquid medium. Other micellar structures are possible and known to those skilled in the art.

In the present invention, once the AB block copolymers comprise a hydrophobic ("water hating") block A and a second hydrophilic ("water loving") block B, such AB block copolymer structures will form micelles with an hydrophilic corona composed of block B in aqueous liquid media or in polar solvents but will also form reversed micelles with a hydrophobic corona composed of block A in more apolar solvents such as an aliphatic oil. In aqueous liquid media, such amphiphilic copolymers are utilised that form spherical aggregates at the concentrations employed.

In the present invention, once the AB block copolymer comprises two hydrophobic blocks A and B differentiated by having different solubility parameters in the same liquid medium, such structures will form micelles with a corona composed of block A in a liquid medium where the block B is less soluble than the block A in the chosen liquid medium or micelles with a corona composed of block B in a liquid medium where the block A is less soluble than the block B in the chosen liquid medium.

The chemistry of the micelles (micellar aggregates) should be such that the micelles will adsorb freely onto a wide variety of particle surfaces. Also, the composition may form micelles preferably having a maximum dimension [diameter in the case of spherical micelles] of from to 3 to 500nm, preferably from 3 to 300nm. The block-copolymer micelle structures most preferably have a maximum dimension [diameter in the case of spherical micelles] of from 10 to 100nm.

In a preferred example, the polymers used in the composition are prepared by controlled living radical polymerisation reactions. Preferably, the block copolymers are prepared by means of controlled living radical polymerisation to obtain narrow molecular weight distribution copolymers. Suitable synthetic routes include but are not limited to: Reversible Addition - Fragmentation chain Transfer (RAFT), Group Transfer Polymerisation (GTP) and Atomic Transfer Radical Polymerisation (ATRP), Activated Regenerated by Electron Transfer (ARGET), Nitroxide-Mediated Polymerization (NMP), ring-opening polymerization and ionic type of polymerization and combinations of techniques where appropriate.

In a further aspect, the micelles may be crosslinked resulting in a more durable coating on the substrate. In the current invention, crosslinking can be described as the physical and/or chemical interaction between chains of the AB diblock copolymer. The crosslinking can take place either in the core of the micelles, in the corona of the micelles and/or between the coronas of two contiguous micelles and the crosslinking may or may not be reversible.

Chemical crosslinking requires the use of a molecule called a crosslinker or crosslinking reagent. Three preferred chemical crosslinking strategies are: (1) crosslinking with a multifunctional organic compound, for example via condensation or addition reactions (such as carboxylic acids with amines; carboxylic acids with hydroxyls; and hydroxyls with isocyanates); (2) ring-opening reactions (such as epoxy groups with amines); and (3) radical initiated crosslinking of vinyl or similar chemical functions (such as those in divinyl benzene and/or di-methacrylates); which can be introduced to the AB di-block copolymers.

A multifunctional organic compound is defined as an organic compound containing two or more functional groups that may react with functional groups described for the AB di-block copolymers used in this invention to form crosslinks. The functional groups in the organic compound may be any that will react with functions described herein for the AB di-block copolymer including but not limited to: amine, hydroxyl, carbonyls such as ketones or aldehydes, carboxyls such as carboxylic acid, isocyanates and sulfhydryl.

Vinyl groups may be introduced to the AB di-block copolymer by using vinyl compounds that also contain a function that will react with functional groups described in this invention for the AB di-block copolymer. Examples of such chemistry include, but are not limited to, amine functionalised vinyl compounds such as amino alkyl methacrylates. Following the introduction of the vinyl chemistry, crosslinking is carried out by radical initiation via thermal or UV curing.

Chemical covalent crosslinks are stable mechanically and thermally, so once formed are difficult to break, whereas physical crosslinks are reversible and the physical crosslinking process may or may not require the use of crosslinking agents. Physical crosslinking occurs when there is the formation of a physical interaction between functional groups located either in the AB diblock copolymer alone or between functional groups located in the AB diblock copolymer and in the multifunctional crosslinker. Techniques include, but are not limited to, dehydrothermal treatment, plasma treatment, hydrogen bonding, ionic interactions and freeze thawing.

Crosslinking (physical and/or chemical) can bring many benefits including, in aqueous-based systems or polar systems, making the hydrophilic (or hydrophobic) corona of micelles more hydrophobic( and making corresponding changes in apolar systems). This provides the opportunity to control the release rate of an active coated with crosslinked micelles.

In the present invention, the block copolymer comprises at least one block that adsorbs to a target surface. The composition may further comprise an adhesion promoter (AP). An AP will generally consist of a polyelectrolyte of opposite potential (charge) to the potential (charge) of the crystal (crystalline particle); in this case the block copolymer micelles coat the AP modified surface. This allows copolymer micelles of similar potential (charge) to the crystal to be deposited on the crystal (through a crystal-AP-block copolymer arrangement).

Also in a composition used in accordance with the present invention the liquid medium may comprise water, water and an organic solvent, an organic solvent or mixtures of solvent, or an organic solvent free from water; wherein the block copolymer is preferably completely dissolved in the liquid medium. To especially but not exclusively encourage reverse micellisation the liquid medium employed will consist of two solvents, one a good solvent for the block-copolymer and a second, less effective, solvent which will cause separation of the block co-polymer from solution and the formation of micelles. This second solvent is generally classed as an apolar solvent.

Generally preferred are polar solvents such as water-miscible organic solvents that can be selected from: C₁₋₆ alcohol (preferably, methanol, ethanol, n-propanol, iso-propanol, n-butanol, tert-butanol or sec-butanol), alkylketones, arylalkylketones, ketoalcohols, cyclic ketones, heterocyclic ketones, ethers (such as tetrahydrofuran), cyclic ethers (preferably ethylene glycol or glycol ethers), esters (preferably ethyl acetate), amides (preferably dimethylformamide) and sulfoxides (preferably dimethylsulfoxide) and combinations thereof. Other powerful solvents, although not water miscible, are aromatic solvents - such as toluene, xylene and the higher homologues and analogues routinely employed as solvents in commercial products, such as Solvesso 100, Solvesso 150. Higher aromatic solvents such as alkyl naphthalenes, for example Solvesso 200 and Solvesso 200ND may be employed as powerful solvents for oil-soluble copolymers that may be employed to form micelles (normal or inverse) in an oil medium. Any solvent used conventionally in agrochemical formulations may be suitable for use in the present invention (for example, cyclohexanone, alkylcyclohexanones, NMP, N-octyl pyrrolidone and C8-C10 fatty acid amides).

Preferred apolar solvents, as opposed to polar solvents, may be selected from but are not limited to: alkanes, preferably pentane and hexane; halogenated solvents, preferably dichloromethane; chloroform, chlorobenzene and fluoroalkanes; and aromatic solvents and combinations thereof. Suitable apolar solvents may also be selected from what are generally classified as oils, such as high molecular weight alkanes, for example paraffinic oil; such as Isopar V and Exxsol D140; alimentary oil such as olive oil, soy bean oil and castor oil and combinations thereof. Conventional ester solvents are also suitable.

When a composition of the present invention comprises a liquid, the ratio by weight of block copolymer to the liquid medium is preferably from 1 : 100,000 to 1 : 1 ; more preferably from 1 : 10,000 to 1 : 2; especially from 1 : 5,000 to 1 : 5; and most preferably from 1 : 5,000 to 1 : 10.

It will also be appreciated by one skilled in the art that the composition according to the present invention may preferably further comprise additional components or auxiliary agents selected from for example but not limited to dispersants, perfumes, biocides, stabilisers, surfactants, wetting agents, emulsifiers, colouring agents, dyes, pigments, UV absorbers, radical scavengers, antioxidants, anti-corrosion agents, optical brighteners, fluorescers, bleaches, bleach activators, bleach catalysts, non-activated enzymes, enzyme stabilizing systems, chelants, coating aids, metal catalysts, metal oxide catalysts, organometallic catalysts, filmforming promoters, hardeners, linking accelerators, flow agents, levelling agents, defoaming agents, lubricants, matte particles, rheological modifiers, thickeners, conductive or non-conductive metal oxide particles, magnetic particles, anti-static agents, pH control agents, preservatives, pesticides (for example, herbicides, insecticides and fungicides), anti-fouling agents, algicides, bactericides, germicides, disinfectants, bio-effecting agents, vitamins, drugs and therapeutic agents and a combination thereof.

We have now found that these micelle structures can be conveniently employed to coat small particulate materials such as organic crystals. Therefore, in a further aspect, the present invention provides a coated particle where the particle is an organic crystalline particle. The chemistry of these applications is thereby incorporated herein. Moreover, the technique is easy to employ and permits high coating weights to be applied over all a surface (including corners and edges of crystals if present). The micelle structures used in the present invention can produce a coating thickness typically up to 50nm in a single pass treatment - very much higher than any other known technique - while maintaining complete stability and non-agglomeration of the coated particle. Multi-coats produce even higher coating weights and thicknesses.

The block copolymers used in the present invention form micellar aggregates typically from 3 to 500nm in size, suitably from 3 to 300nm. Aggregation number is controlled by the chemistry of the block copolymer in terms of absolute chemistry, charge, molecular weight and the solution conditions under which the micelle is formed. Typical aggregation numbers for such a block copolymer micelle can be of the order of 100 molecules. Therefore suitably the micelles are present as micellar aggregates which each comprise from 10 to 1000 molecules. Typical molecular weights of block-copolymers used in the present invention are from 3000 to 100000Dalton but are specified within the chemistry.

Block copolymer micelles can be simply employed by adding to a dispersion of a particle in a carrier liquid and allowing to equilibrate. Confirmation of coating can be obtained by SEM observation and quantitative data by analysis of a sample for total active material content (where an active material is coated). Other techniques to induce micelle formation (such as pH shift, temperature, solvent exchange and dilution) can all be suitably employed. As an alternative process, a drying technique to remove either a solvent or to induce a chemical change - such as loss of ammonia in a drying process - can be employed.

To coat a particle, the process can be simply achieved by adding block copolymer micelles (or inducing block copolymer micelles to form) to a dispersion of a particle in a carrier liquid. Therefore in a further aspect, the present invention provides a process for preparing a particle as described herein comprising the steps of (a) forming micelles of the copolymer; and (b) mixing the micelles with the crystalline particle.

As further embodiments, we have found that if the particle dispersion is pre-treated with an adhesion promoter, which is not a copolymer micelle, then improved deposition of block copolymer micelles is achieved.

In one embodiment the adhesion promoter is a polyelectrolyte which is a homopolymer selected from, but not limited to: poly(diallyldimethylammonium chloride) (PDADMAC), poly(sodium styrene sulfonate) (PNaSS), poly(methacrylic acid sodium salt), poly(acrylic acid sodium salt), poly(vinylpyridinium salt) and poly(alkylammonium salt). In a further embodiment, the adhesion promoter can be a block copolymer micelle in its own right such that a double coating of oppositely charged micelles is achieved. In a still further embodiment, it is possible to build up significant multiple layers of micelles by sequential treatment. It is possible to contemplate an amphiphilic block copolymer with amphoteric properties such that by simple pH switching, several layers of the same copolymer can be induced to be deposited. In order to modify significantly a particle surface, the number of micelles coating an individual particle has to be large. Usually there will be at least a 10-fold greater number of micelles per coated particle (and usually significantly greater).

Certain products of the invention comprise a particulate material covered with a coating of block copolymer micelles (including, uniquely, edges and corners as well as faces). A key aspect of the present invention is the ability to provide good coverage [and protection] for sharp features such as edges and, particularly, corners of crystals. Therefore suitably a particle according to the present invention is coated by at least 10 micelles. More preferably, the particles according to the present invention are entirely coated by the micelles.

The copolymers according to the present invention have been found to form complex and large micelles in solution.

The nature of the micelle (in terms of whether the core comprises a hydrophobic structure and the corona a hydrophilic structure; or whether it is a reverse [also referred to as 'inverse'] micelle comprising a hydrophilic core and a hydrophobic corona) is dictated by both the chemistry of the block copolymer and the solvent environment in which the block copolymer is formulated. In some chemistries, the amphiphilic character can be introduced by having two blocks wherein one block is significantly more hydrophobic than the second block, this inducing a differential solubility in the blocks and thereby inducing an amphiphilic structure that enables the formation of micelles. Importantly, to form micelles (that is aggregates formed by molecules with an amphiphilic character) in a liquid medium, one block of the copolymer should be poorly soluble in the liquid to drive the formation of aggregates of the molecules. The structures of the aggregates are dependent on the copolymer concentration and the exact nature of the copolymer molecules as well as the nature of the liquid environment (for example, liquid type and temperature). In the present invention copolymers are utilised that preferably form spherical aggregates at the concentrations employed but other micelle shapes will also work with the present invention.

Micelles are generally composed of two defined regions within their structures; a central "core" where all the hydrophobic parts of a surfactant are aligned together and an external "corona" where all the hydrophilic parts of a surfactant are aligned. In a normal micelle, the core is the more hydrophobic region and the corona is the more hydrophilic region. In a reverse micelle, the opposite is true where the core is the more hydrophilic region and the corona is the more hydrophobic (and in this context, a more hydrophilic region does not have to be water soluble, just sufficiently more hydrophilic than the hydrophobic part of the molecule to induce phase separation into such micelle structures. The coronal chemistry for the micellar aggregates is such that the micelles will adsorb freely onto a wide variety of particle surfaces.

The AB block copolymer may suitably take any form selected from: linear block copolymer (diblock, triblock or multiblock), miktoarm copolymer (star copolymer), ladder (H-shaped) copolymer, graft and comb (co)polymer; preferably it is a linear block copolymer.

The distribution of component monomers within each copolymer block is selected from the form of homo, random, gradient, alternative, block, graft and comb (co)polymers; that is, any type of copolymer structure which will lead to a segregation of copolymer in the liquid medium as organised aggregates.

It is preferred that the block copolymer is selected from the group comprising: AB blocks, ABA blocks and ABC blocks.

In accordance with the present invention the block copolymer comprises at least one block that absorbs to a target surface. The composition may further comprise an adhesion promoter. Also, the composition must form micelles and the micellar aggregate structures in the composition preferably have a maximum dimension [or diameter for spherical micelles] of from 3 to 300nm. The block-copolymer micelles most preferably have a maximum dimension [or diameter for spherical micelles] of from 10 to 100nm.

In a preferred embodiment, the polymers used in the composition are prepared by a controlled living radical polymerisation reaction.

In a composition used in accordance with the present invention the liquid medium may comprise water, water and organic solvent, an organic solvent or mixture of solvents or an organic solvent free from water; wherein the block copolymer is preferably completely dissolved in the liquid medium before micelle formation.

Examples of small materials to be coated are objects that need to be protected from their environment, for example water soluble organic crystals that may be otherwise incompatible in an aqueous formulation and particles which may react with the other ingredients of the formulation causing an increase of viscosity and decrease in the shelf life of the formulation.

Products of utility may be an agrochemical, laundry chemicals, cosmetics, food additives, paint and coating additives, biocides for paints, pharmaceutical and other particles that find utility in various fields. The novel coating, produced by micelle-forming polymers, finds utility in a variety of ways. The coated particle can now be more effectively targeted for adhesion to a substrate by selection of the block copolymer, as in targeting a specific substrate in agriculture (such as an insect cuticle, leaf surface or fungal pathogen) or in pharma (for delivery to a specific target organ or protection of an agent for delivery through the mammalian stomach for selective and protected delivery later in the digestion system) or in laundry (for release of an agent at the appropriate point in the wash cycle). Moreover, the effectively coated particles confer greater colloidal stability on systems, allowing greater and improved stability when mixed with other components.

Further suitable applications include, without limitation: sustained release and controlled release usages, for example: in the pharmaceutical field, for example acid resistant structures (oral delivery past low pH in the stomach), protection of labile actives, pseudo-zero order release through the micelle layer and Ostwald-ripening resistant formulations; cosmetics; perfumes, for example slowing down evaporation of top-notes or sustained release and minimising overpowering odours; particles having affinity for cellulose and trapped on textile surface during laundering; flavours, for example light stabilised to prevent oxidation; self-healing coatings, for example particle induced to burst to release a resin that repairs damage; carbonless copy paper; novel, double taste and texture food, for example particle which dissolves in the mouth and releases a new taste; pressure sensitive adhesives; sealants; nutrition (for example increased bioavailability of complex molecules and protection of sensitive molecules such as vitamins, probiotics and other food additives); toner inks with photosensitivity or thermal sensitivity; textile coatings, for example, for altering permeability properties; antifouling coatings; surface protective coatings, for example, for improving scratch or abrasion resistance; and construction materials, for example wall-boards, plasterboards and cements.

It is well known that chemical incompatibility between different components in liquid laundry formulations can cause instability in these formulations. In particular, laundry bleach activation agents such as, but not limited to, tetraacetylethylenediamine (TAED) that are widely used in powder laundry formulations are incompatible with liquid laundry detergents. Bleach activation agents, precursors and catalysts tend to be unstable in many liquid formulations and although the surfactants in the liquid formulation are stable they can react with bleach or bleach activator chemicals or catalysts or derivatives of them. One solution is to add a solid form bleach activator as a separate dose to the liquid laundry detergent, but this is inconvenient for the consumer. The present invention provides a means of protecting the solid bleach activator from interaction with water and other liquid detergent components to enable a stable liquid detergent to be formulated.

The sustained release of biocides and anti-fouling agents is of commercial interest to the paints and coatings industry and in particular for marine applications. One example of a biocide that has been employed as an antifouling agent for marine use is DCOIT (4,5-dichloro-2-n-octyl-3(2H)-isothiazolone). This active has a low solubility in sea water which is extremely desirable, however in solvents used in paint formulations such as xylene, it is extremely soluble. This means that it is likely to react with the paint binders within the formulation and may increase the paint viscosity or induce plasticizing of the paint. Marine paint manufacturers will benefit from a biocide that improves in-can stability of the paint whilst incorporating sustained release of the active after application onto the marine vessel. The present invention provides a means of protecting the biocide from the other active ingredients in a paint formulation and provides a means of sustained release in sea water.

Safe delivery of active pharmaceutical ingredients (APIs) to the intended target site within a mammalian body is major area of both commercial unmet need and scientific research. In many cases the API needs to be protected from interaction with its environment in order to prevent unwanted chemical reaction or biological use of the active at the wrong site within the body or at the wrong rate. One solution to this problem is to formulate the API into a tablet and to add a protective or enteric coating to the tablet. This can be sub-optimal for a number of reasons including patient preference for non-tablet formulation and the potential risk of over-dosing [if the enteric coating fails]. The present invention enables individual crystals of API to be coated enabling formulation into a capsule rather than a tablet and minimizing the risk of over-dosing [as the coating would need to fail multiple times on individually coated API crystals rather than only once on the tablet].

Non Steroidal Anti Inflammatory Drugs (NSAIDs) such as ibuprofen and diclofenac are limited in their administration because at higher doses, side effects such as gastric erosion, thrombasthenia, thrombocytopenia and fluid retention may become severe.

Vitamin C is also known as ascorbic acid, ascorbate and ascorbate monoanion. It is the enolic form of an α-ketolactone. Vitamin C works physiologically as a water soluble antioxidant by virtue of its high reducing power. It acts as singlet oxygen quencher and it is capable of regenerating vitamin E. Vitamin C is called an antioxidant because of its ability to quench or stabilise free radicals that otherwise may lead over time to degenerative diseases, including cancer, cardiovascular disease and cataracts.

Ascorbic acid properties are impaired by its high reactivity, and hence, poor stability in solution, which can result in heavy losses during food processing. It can be degraded rapidly in the presence of oxygen or by free-radical mediated oxidative processes. The processes are strongly catalysed by transition metal ions, especially iron and cooper, leading to rapid destruction of the ascorbate. Oxidation is also accelerated at neutral pH and above. Destruction may occur due to the presence of enzymes, such as ascorbate oxidase and ascorbate peroxidase.

The food industry may employ microencapsulation to produce foods which are more nutritionally complete. The properties of microencapsulated nutrients will allow the food processor greater flexibility and control in developing foods with high nutritional value. Ascorbic acid is added extensively to many types of food products for two quite different purposes: as a vitamin supplement to reinforce dietary intake of Vitamin C, and as an antioxidant, to protect the sensory and nutritive quality of the food itself.

The present invention enables individual crystals of ascorbic acid or other food supplements to be coated for application in the food industry as fortification. Coated particles may be incorporated in dry form into cake mixes, puddings, gelatine desserts, chewing gum, milk powder, jellies, pet foods or breakfast cereals, in short, into products with low water activity.

A composition according to the present invention may suitably be an agrochemical formulation; the agrochemical formulation may comprise an agrochemical active ingredient (such as a fungicide, herbicide, insecticide or plant growth regulator) or it may comprise an adjuvant which is used to enhance the bioperformance of an agrochemical [either in the same formulation as the adjuvant or to be applied from a separate formulation]. The composition can be in the form of a concentrate which is diluted or dispersed in a spray tank prior to use, although ready-to-use compositions can also be made. The final dilution is usually made with water, but can be made instead of, or in addition to, water, with, for example, liquid fertilisers, micronutrients, biological organisms, oil or solvents. The compositions may be chosen from a number of formulation types, many of which are known from the Manual on Development and Use of FAO Specifications for Plant Protection Products, 5th Edition, 1999. These include dustable powders (DP), soluble powders (SP), water soluble granules (SG), water dispersible granules (WG), wettable powders (WP), granules (GR) (slow or fast release), dispersible concentrates (DC), suspension concentrates (SC), capsule suspensions (CS; in which case, the particle is a microcapsule) and seed treatment formulations. The agrochemical formulation may be used to control or combat a pest [examples of agricultural pests include unwanted plants (weeds), insects and fungi].

As further embodiments, micelles comprise a core and a corona that are chemically different. This difference can be exploited for further benefits. The micelle core can be selectively loaded with a component that dissolves (or can be dissolved by a suitable solvent) in the core chemistry. For example, the application of a photostabiliser by such a technique (by incorporating the photostabiliser into the micelle core which is then coated onto the crystal surface) will improve the ability to stabilise sensitive chemistry against photolytic degradation. Soil mobility of crystalline particles of a pesticide can similarly be enhanced by coating a stable polymer micelle onto the crystal surface (optionally in combination with added specific surfactants that can promoted improve soil mobility). In some situations, pesticides can induce a phytotoxic response (in cotton for example) due to too rapid a photolytic degradation. Coating crystals in this manner with a polymer micelle containing a photostabiliser could reduce the rate of degradation.

Therefore, in another aspect, the present invention provides a crystalline particle as described herein where the cores of the micelles contain a chemical, which may be a photoprotectant, a biologically active compound or an adjuvant (for example an adjuvant for improving or controlling the bioperfomance of an agrochemical).

Furthermore, the coated crystalline particle may be a biologically active compound [for example, an agrochemical] whilst the micelle core may be loaded with a second biologically active compound [for example, an agrochemical]. Alternatively two or more different biologically active compounds [for example, agrochemicals] may be mixed together as coated particles according to the present invention in such a manner that the micellar coatings overcome any potential incompatibility problems [for example, physical or chemical incompatibility].

In a further aspect, the ability to coat such block copolymer micelle systems onto a substrate provides an elegant procedure to prepare mixed products by coating a polymer micelle containing a first active onto a crystal surface of a second active (with the option of further actives being in a dissolved or other dispersed state). Further, such coated polymer systems could then be applied to relevant surfaces that could require protection against attack (such as wood) or onto surfaces where a long lasting barrier might be required (such as to prevent ingress of termites, ants or spiders or to prevent fungal growth in sensitive situations - for example, fungicides in/on wallboards).

Further, non-limiting crop protection applications include particle coating leading to: reduced antagonism by altering the relevant availability of two or more active ingredients, triggered release potential - triggers may be pH, light, water, enzymes - and alteration of release profile. These release rate alterations may be possible not only in the products of the invention but also when subsequently applied (for example to seeds - triggered release from seeds by coating technology - triggers can be pH, light, water or enzymes. The size range of particles to be coated can vary enormously. Where the particle is an organic crystal, the size range can usefully be from 10nm to 500micro, preferably 500nm to 100 micron (although technical material greater than 500micron could be also coated and employed in some utilities (such as pharma) in a pre-granulation stage to protect a material). The size may be defined to be the largest dimension of the particle. Accordingly, in a further aspect of the present invention, the largest dimension of the particle is from 10nm to 5mm. When the crystal size is small, the micelle size chosen to coat the particle has to be even smaller. Where the particle is a granule (or a spray-agglomerated granule) the size can vary from about 50micron to a few millimetres.

Use of this technology can be adapted to coating poorly soluble particles such as pharmaceutical actives including but not limited to griseofulvin, troglitazone, felodipine and ketoconazole (which each have a very low aqueous solubility and a slow dissolution rate) with a hydrophilic polymer system. This is a convenient method of increasing the rate of solubility as the system benefits from a higher surface area and reduced surface/interfacial tension.

The present invention is illustrated by the following examples.

### Example 1: Preparation of Polymers and Block Copolymers.

### 1A. Preparation of Block Copolymers having a hydrophobic block A and a hydrophilic block B.

The copolymers described in this example have a hydrophobic block. This block can comprise one or more monomers, for example; styrene and styrene derivatives, methacrylate and derivatives such as butyl methacrylate (BuMA), trifluoro ethyl methacrylate (TFEMA), ethyl hexyl methacrylate (EHMA), methyl methacrylate (MMA) and propylene oxide (PO). Those skilled in the art will appreciate the synthesis described in this example is not limited to the monomers listed here.

For the polymers described in the current example, the hydrophilic block is composed of methacrylic acid (MAA) or dimethylamino ethyl methacrylate (DMA) but those skilled in the art will understand that other monomers leading to a hydrophilic block can also be used. The copolymers used herein were produced according to the protocol described in the patent applications WO08071957 and WO10038046. The block copolymers may be prepared by means of controlled living polymerisation techniques, such as group transfer polymerisation (GTP), atomic transfer radical polymerisation (ATRP), nitroxide mediated polymerisation (NMP) and activated regenerated by electron transfer (ARGET) or activated generated by electron transfer (AGET) that can synthesize well-defined homopolymers and block copolymers. In this example, in addition to structures described in the patent applications WO08071957 and WO10038046, new copolymer structures were produced by Reversible Addition-Fragmentation Chain Transfer (RAFT) polymerization using the RAFT agent, 2-cyanoisopropyl dithiobenzoate (CPDB). Whilst the current example prepares the block copolymer using the RAFT agent, CPDB, those skilled in the art will appreciate that other RAFT agents may be used.

In addition to controlled radical polymerization, in the case of heterocyclic monomers such as propylene oxide, ring-opening polymerisation techniques can be used. Examples of the composition of new prepared copolymers are given in Table 1.2.

### RAFT synthesis of Poly(BuMA-block-MAA) copolymer : P(BuMA-b-MAA)

A series of poly[BuMAₓ-*b*-MAA_{y}] copolymers were prepared by RAFT polymerization using CPDB as chain transfer agent, azobisisobutyronitrile (AIBN) as initiator, and propan-2-ol (IPA) as a solvent. The synthesis was a two step process. First, the hydrophobic block (BuMA) was synthesised, then the synthesis of the hydrophilic block (MAA) was initiated from the PBuMA homopolymer.

### a) Synthesis of the Hydrophobic block PBuMA.

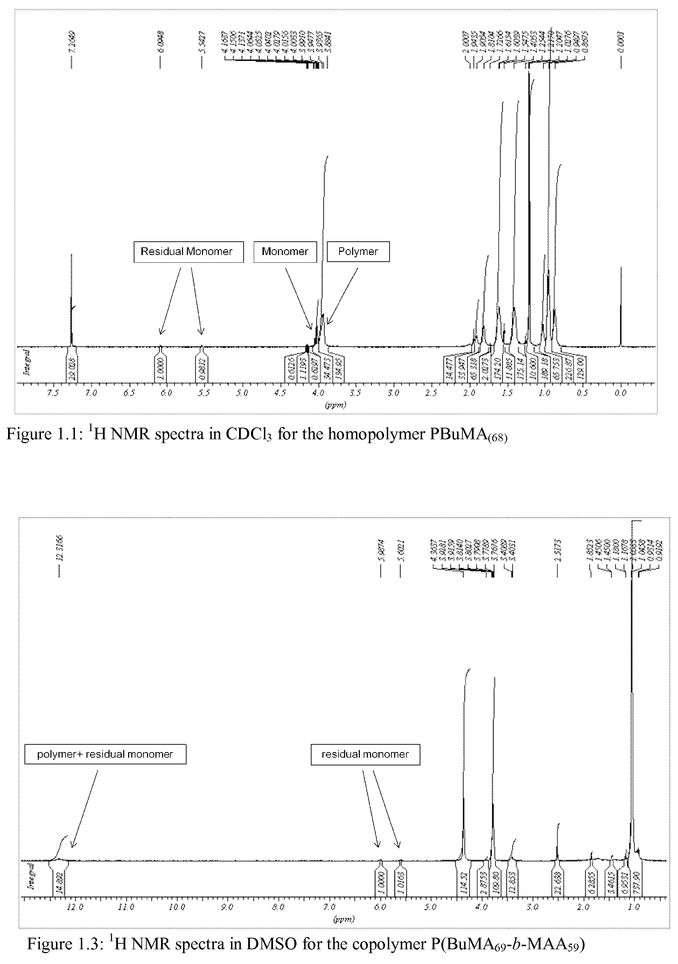

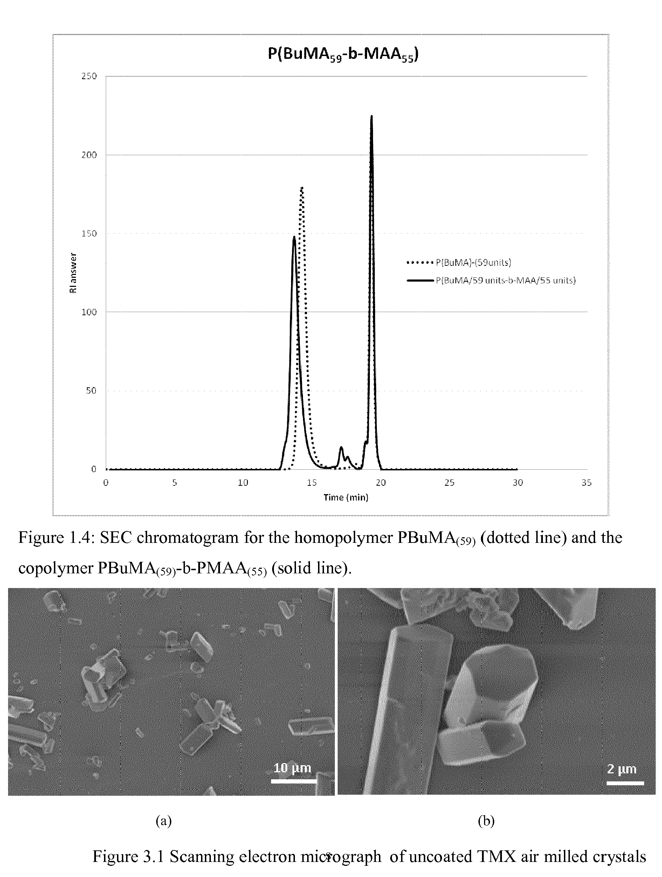

BuMA (15g, 105mmol, 69eq), CPDB (0.37g, 1.51mmol, 1eq), AIBN (0.12g, 0.75mmol, 0.5eq), and IPA (solvent, 6.33g, 105mmol) were added in a two necked flask containing a magnetic stirrer equipped with a cooling column. The mixture was degassed by nitrogen bubbling and heated at 90°C in a thermostatically controlled oil bath under a nitrogen atmosphere. The reaction was left under stirring for a minimum of 2hour 30minute (in this example 2h45min). A sample of the crude mixture was withdrawn and analysed by size exclusion chromatography (SEC - See Figure 1.4), and by ¹H NMR (CDCl₃ - See Figure 1.1). A conversion of 98.3% was determined by ¹H NMR in CDCl₃, hence the resultant product was P(BuMA)ₓ homopolymer where x = 68.

### b) Synthesis of the hydrophilic block from the hydrophobic block

30 minutes before the end of the first synthesis, MAA (7.78g, 90.4mmol, 59.9eq), AIBN (0.12g, 0.75mmol, 0.5eq) and IPA (solvent, 36.2g, 603mmol) were added in another flask containing a magnetic stirrer. The mixture was degassed by nitrogen bubbling. At the end of the first synthesis (in the current example 2h45min), the thermostatically controlled oil bath was removed to stop polymerisation. The mixture containing the second monomer was then transferred into the initial two necked flask via cannula. This flask was heated again at 85°C in the thermostatically controlled oil bath (equipped with a cooling column) under nitrogen atmosphere to achieve the preparation of the second block of copolymer. After a minimum of 2h30min (in this example 2h45min) a sample of the crude mixture was withdrawn and analysed by ¹H NMR (DMSO - See Figure 1.3) and SEC (Figure 1.4).

A conversion of 93% was measured by ¹H NMR in DMSO. The resultant product was determined as P(BuMAₓ-*b*-MAA_{y}) copolymer where x = 68 and y = 55. Other P(BuMAₓ-*b*-MAA_{y}) polymers were prepared with x=59 and y=54 and with x=127 and y=51. The generic structure of the corresponding P(BuMAₓ-*b*-MAA_{y}) copolymers is given below in Formula 1.2:

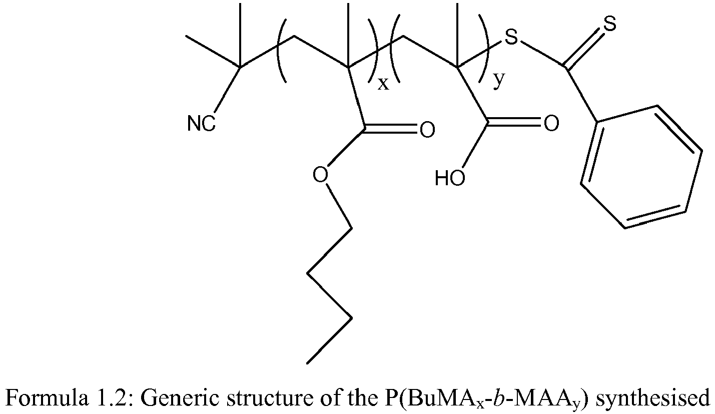

The P(BuMAₓ-*b*-MAAy) copolymers can also be prepared by NMP, ATRP, GTP or indirect anionic polymerization. *Characterisation*
Size exclusion chromatography (SEC) was used to determine the number-average molar mass (Mₙ) and thus demonstrate the increase of molar mass due to the addition of the second block during the RAFT polymerisation. SEC was also used to determine the polydispersity index (PDI= M_{w}/Mₙ, where M_{w} is the weight-average molar mass) of the polymers and copolymers, a low PDI being necessary to achieve regular micelles.

The samples were injected in the SEC equipment. (2 PL gel 5 Micron Mixed-c columns) Analysis was performed as described below
- The eluent was composed of THF (elution flow rate: 1 mL/min, run time: 30 min).
- The calculation (for data analysis) was made with a calibration curve based on poly(methyl methacrylate).
- Before injecting the polymer samples containing methacrylic acid units, a methylation reaction was performed to convert the acid groups into methyl esters, using trimethylsilyldiazomethane as methylating agent, in order to solubilise the polymers in THF to perform the analysis.
- The samples (20mg) were dissolved in the eluent and then filtered with a 0.2µm PTFE filter into the SEC vials.

An example of a SEC chromatogram is given in Figure 1.4. The SEC chromatogram of the first block of P(BuMA) and the chromatogram of the copolymer P(BuMA-*b*-MAA) are represented. The observed shift of the chromatogram is consistent with an extension of chains between both steps.

**Table 1.1: Indication of polydispersity index (PDI) obtained by SEC for some copolymers described in Table 1.2 and 1.3.**

| Copolymer | PDI - block 1 | PDI - block 2 |
|---|---|---|
| P(BuMA₅₉-b-MAA₅₅) | 1.13 | 1.21 |
| P(BuMA₁₂₇-b-MAA₅₁) | 1.17 | 1.28 |
| P(BuMA₃₀-b-DMA₁₀₀) | 1.16 | 1.42 |
| P(MMA₁₅-b-DMA₅₅) | 1.32 | 1.20 |
| P(TFEMA₅₂-b-MAA₂₉) | 1.23 | 1.50 |

Proton Nuclear Magnetic Resonance (¹H NMR) was used to determine the conversion of each polymerisation and accordingly the calculated degree of polymerisation (in number:
DPₙ) for each block.
   ¹H NMR was performed with a 500MHz apparatus (Bruker), in CDCl₃ for the homopolymer, and in DMSO for the copolymer.
   NMR spectra, with the location of the monomer and polymer peaks, are given in Figures 1.1 and 1.3.

### Preparation of other copolymers by RAFT synthesis.

Various block copolymers were synthesised. The hydrophobic block was obtained from styrene and various methacrylate based monomers such as TFEMA and EHMA. The hydrophilic block was composed each time of MAA, HEMA or DMA units.

The method described above for the synthesis of P(BuMAₓ-*b*-MAA_{y}) was used, which led to the successful synthesis of P(TFEMAₓ-*b*-MAA_{y}), P(EHMAₓ-*b*-MAA_{y}), P(MMAₓ-*b*-MA_{y}), and P(BuMAₓ-*b*-MA_{y}). The conversion rates, block sizes and reaction times are given in Table 1.2:

**Table 1.2**

| | Block 1 | | | Block 2 | | |
|---|---|---|---|---|---|---|
| Copolymer | DPn th (units) | Conv. / time | DPn exp x = | DPn th (units) | Conv. / time | DPn exp y = |
| P(BuMAₓ-b-MAA_{y}) | 69 | 98%, 2h45 | 68 | 59 | 93%, 2h45 | 55 |
| P(BuMAₓ-b-MAA_{y}) | 136 | 93%, 2h45 | 127 | 60 | 85%, 3h05 | 51 |
| P(TFEMAₓ-b-MAA_{y}) | 60 | 98%, 2h15 | 59 | 60 | 84%, 3h15 | 50 |
| P(BuMAₓ-b-DMA_{y})ⁱ⁾ | 30 | 85%, 3h00 | 26 | 100 | 79%, 3h00 | 95 |
| P(MMAₓ-b-DMA_{y})ⁱ⁾ | 15 | 92 %, 2h30 | 14 | 55 | 98 %, 6h40 | 54 |
| P(EHMAₓ-b-MAA_{y}) | 60 | 98%, 3h15 | 59 | 60 | 88%, 2h35 | 53 |
| P(Styₓ-b-HEMA_{y}) ⁱⁱ⁾ | 55 | 40%, 5 h 00 | 22 | 110 | 55 % 18 h 00 | 61 |

Table 1.2: Synthesis and composition data according to ¹H NMR BuMA: butyl methacrylate; MAA: methacrylic acid; TFEMA: trifluoroethyl methacrylate; DMA: N,N-dimethylaminoethyl methacrylate; MMA: methyl methacrylate; EHMA: 2-ethyl hexyl methacrylate; Sty: styrene; HEMA: 2-hydroxyethyl methacrylate; Conv.: conversion given in %; DPn th: degree of polymerisation targeted; DPn exp: degree of polymerisation calculated;
i) Copolymers with a DMA block can be classified as well as amphiphilic copolymers when they are dispersed in low pH aqueous solution.
ii) Synthesis performed in DMF instead of IPA.

### 1b : Preparation of Block Copolymers having two hydrophobic blocks A and B.

Block copolymers having two hydrophobic blocks were prepared following the same procedure as used for the preparation of P(BuMAₓ-*b*-MAAy) copolymers as described in the Example 1 section 1a. Examples of the structures prepared by this method are listed in Table 1.3:

**Table 1.3**

| | Block 1 | | | Block 2 | | |
|---|---|---|---|---|---|---|
| Copolymer | DPn th (units) | Conv. / time | DPn exp x = | DPn th (units) | Conv. / time | DPn exp y = |
| P(LMAₓ-b-EHMA_{y}) ⁽ⁱ⁾ | 35 | 95.3%, 1h30 | 33 | 106 | 100%, 2h05 | 106 |
| P(EHMAₓ-b-ODMA_{y}) ⁽ⁱ⁾ | 42 | 95.2 % 1h15 | 40 | 70 | 100 %, 19h00 | 70 |

Table 1.3: Synthesis and composition data according to ¹H NMR; EHMA: 2-ethyl hexyl methacrylate; LMA: lauryl methacrylate; ODMA: octadecyl methacrylate; Conv.: conversion given in %; DPn th: theoretical degree of polymerisation; DPn exp: the-experimental degree of polymerisation measured;
(i): Synthesis performed using RAFT in toluene

### Example 2: Demonstration of micelle formation in an aqeous system or polar solvent

Micellar aggregates can be formed from the copolymers of Example 1 by a number or routes. One such route is described below. Size distribution measurements using a Malvern Nano Zetasizer were performed to demonstrate micelle formation for solutions of copolymers in water-based mixtures or in organic solvents such as toluene, ethyl acetate, dodecane, hexane, Exxsol D140, Solvesso 200ND and Isopar V.

Production and characterisation of micelles
1. The copolymer was dissolved in a good solvent with the assistance of gentle agitation (for example using a magnetic stirrer set on low for 1 hour).
2. When the polymer had dissolved, a second solvent was added drop-by-drop until it reached a large enough quantity that it became the continuous phase. The second solvent was chosen to be a poor solvent for one of the copolymer blocks and a good solvent for the other block, thus inducing the formation of micelles.
3. To ensure equilibrium was reached, the mixture was gently agitated for 2 hours, using a magnetic stirrer set on low. At the conclusion of this period, stable micelles were formed. The following sections detail the precise conditions used to form micelles from the copolymers of Example 1 in a range of solvents.

Table 2.1 shows the structures of the copolymers used for this experiment, the concentration of the micellar solutions and recorded micelle size in solution.

### Aqueous systems and polar solvents

1. The copolymer was dissolved as a 1wt% solution in either water or ethanol. As described in patent applications WO08071957 and WO10038046, ethanol was used (at 8wt%) if the copolymer did not dissolve directly in water or other organic solvents.
2. A second solvent, in this case water, methanol or ethyl acetate was added in a drop by drop manner until a concentration of 0.05-1wt% was reached.
3. The solution was gently agitated for at least 2 hours using a magnetic stirrer set on low in order to allow the micelles to stabilise in solution.

To ensure an accurate measurement, using the Malvern Nano Zetasizer it is important to have the correct concentration for a given copolymer solution. The optimum concentration range for the examples herein was shown to be 0.05-1wt%. The size distribution measurements in Table 2.1 shows that the copolymers form micelles, since the minimum diameter is 6 - 11nm and if the copolymers were present as unimers, the diameter would be less than 5 nm.

**Table 2.1**

| Hydrophobic Block | | Hydrophilic Block | | Characterisation Zetasizer | |
|---|---|---|---|---|---|
| Monomer | No of units | Monomer | No of units | Concentration (wt %) | Diameter range (nm) |
| BuMA i) | 15 | MAA | 120 | 0.25 | 30-90 |
| BuMA i) | 59 | MAA | 54 | 0.5 | 6-11 |
| BuMA i) | 127 | MAA | 51 | 0.25 | 20-50 |
| TFEMA i) | 20 | MAA | 140 | 0.05 | 30 - 50 |
| EHMA i) | 29 | MAA | 49 | 0.5 | 30-60 |
| Styrene ii) | 22 | HEMA | 61 | 1 | 8-30 |
| TFEMA iii) | 20 | MAA | 140 | 0.05 | 96-118 |
| EHMA iii) | 29 | MAA | 49 | 0.05 | 102-108 |

Table 2,1 Zeta sizer data obtained from copolymers dispersed in water-based and polar media. i) Measurements made from aqueous solution; ii) Measurements made from methanol solution; iii) Measurements made from ethyl acetate solution. Measurements collected using a Malvern Zetasizer.

The data in Table 2.1 demonstrate that copolymer micelles can be formed in a range of polar solvents, with the size of the micelles ranging from 6 to 108nm.

### Example 3. Coating Crystal in a aqueous system

### 3.a. Layer by Layer Coating of a crystal, with one layer of homopolymer containing cationic charges, and one layer of negatively polarised copolymer

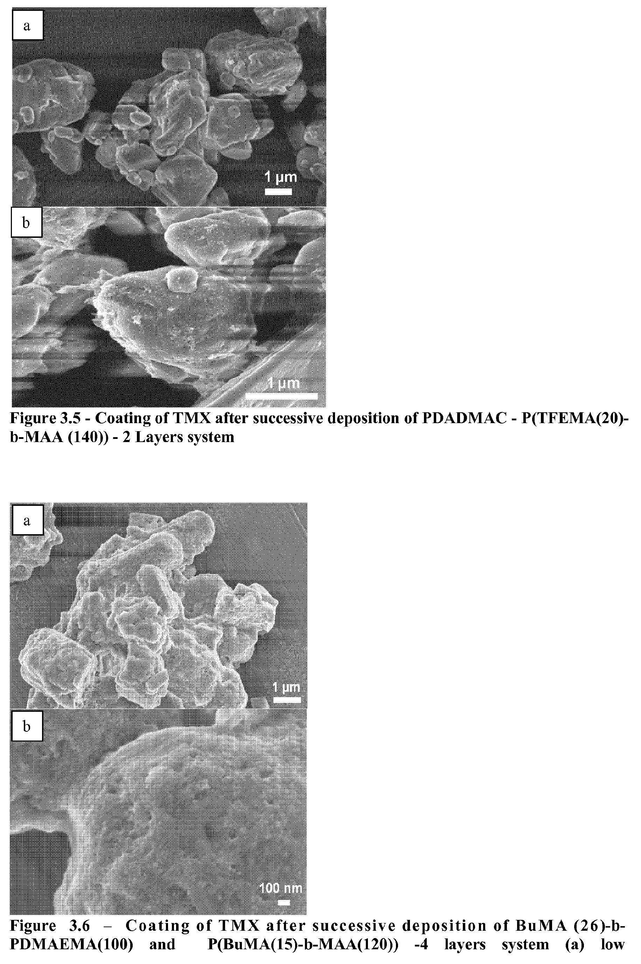

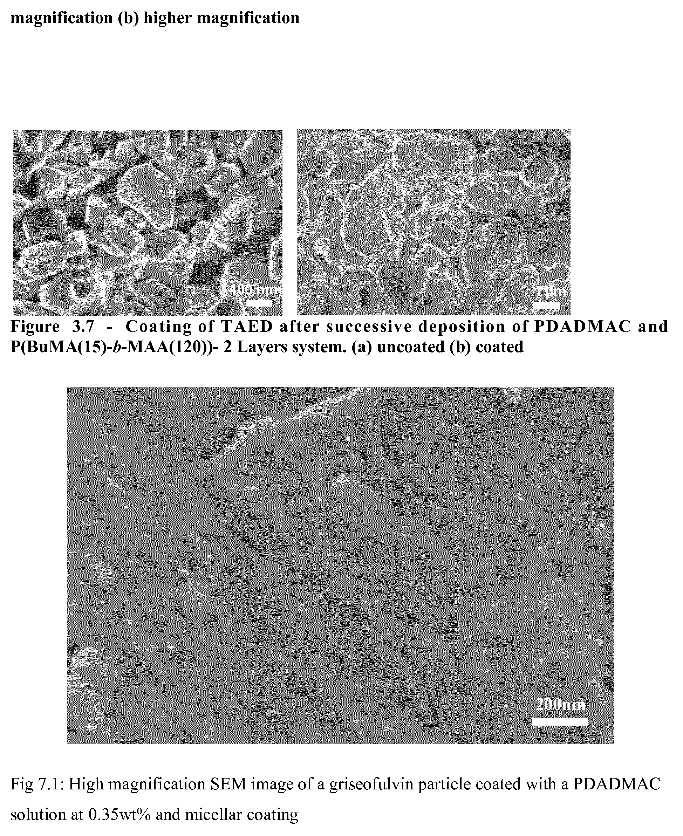

Thiamethoxam crystals (TMX) with a size distribution of approximately 2.5 - 5µm (Figure 3.1) were coated with two layers of copolymers.

First, a layer of poly(diallyldimethylammonium chloride) (PDADMAC) homopolymer containing cationic charges was applied on the crystal. Then, a layer of negatively polarised copolymer, P(BuMA(15)-*b*-MAA(120)) was applied following the protocol described below.

The coating protocol required that the crystal to be coated remained dispersed in the liquid medium. In this example, TMX was coated in water. As TMX is soluble in water up to 4.1g/litre, a saturated stock solution of TMX was prepared at a concentration much higher than 4.1 g/litre. The experimental procedure is detailed below:
1. 1g of negatively charged TMX particles was placed in 10ml of TMX saturated stock solution.
2. A 10ml PDADMAC solution (0.35wt%) was added to the TMX solution.
3. The sample was tumbled for 30minutes.
4. The sample was then centrifuged for 2minutes at 2000rpm in order to deposit the particles onto the bottom of the tube.
5. Following centrifugation 15ml of the supernatant was removed.
6. This was replaced by 15ml of TMX saturated stock solution.
7. The sample was tumbled for 30 minutes.
8. The sample was then centrifuged for 2 minutes at 2000rpm in order to deposit the particles onto the bottom of the tube.
9. Following centrifugation, 15ml of the supernatant was removed
10. The solution concentration was then brought back up to 10ml by adding 5ml of TMX saturated stock solution.
11. 10ml of a 1wt % solution of P(BuMA(15)-b-MAA(120)) was added to the TMX solution.
12. The sample was tumbled for 30 minutes.
13. The sample was then centrifuged for 2 minutes at 2000rpm in order to deposit the particles onto the bottom of the tube.
14. Following centrifugation 15ml of the supernatant was removed.
15. This was replaced with 15ml of TMX saturated stock solution.
16. The sample was tumbled for 30 minutes.
17. The sample was then centrifuged for 2 minutes at 2000rpm in order to deposit the particles onto the bottom of the tube.
18. Following centrifugation 15ml of the supernatant was removed.
19. The solution concentration was then brought back up to 10ml by adding 5ml of TMX saturated stock solution.

Note: The pH of each solution was adjusted to, and maintained at pH9 using a 35wt% ammonia solution or a 0.1M potassium hydroxide (KOH) solution in water.

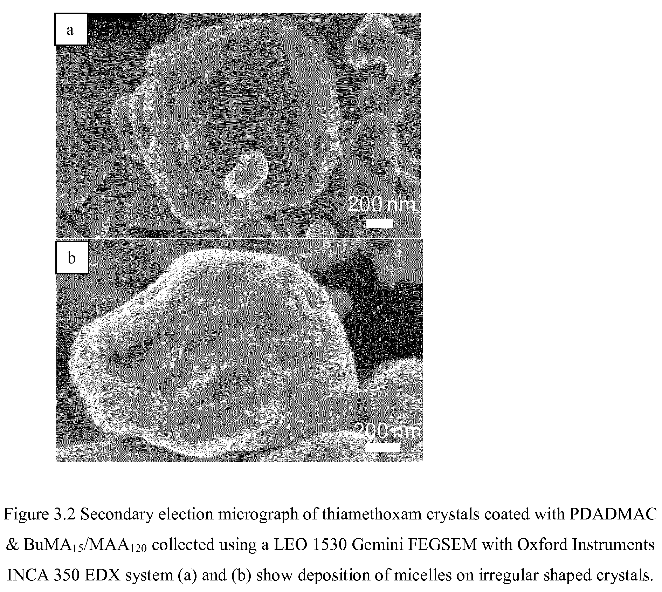

A sample of the mixture in step 8 was withdrawn and analysed by Scanning Electron Microscopy. Pictures of this final coating are shown in Figure 3.2(a & b)
Figure 3.2 clearly shows micellar deposits on all faces, corners and edges of the TMX crystals as indicated by the uneven topography and rounded edges in comparison to the uncoated TMX crystals shown in Figure 3.1.

### 3.b. Layer by Layer Coating of a crystal, with one layer of copolymer containing cationic charges, and one layer of negatively polarised copolymer

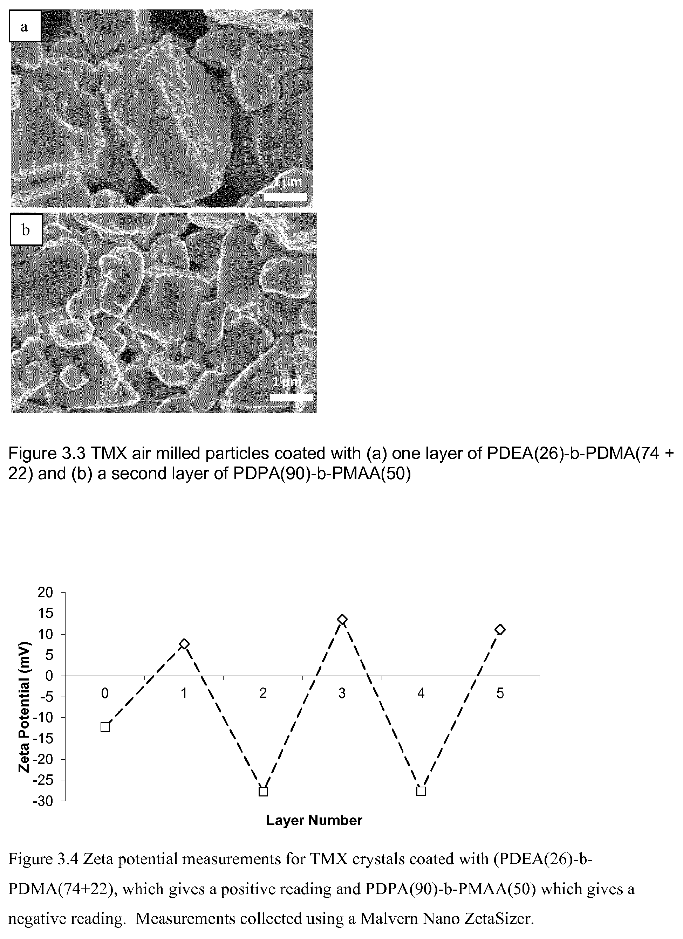

The adhesion promoter PDADMAC of example 3a can be replaced by cationically charged copolymers and by repeating the process of 3a in order to deposit a double layer of copolymer micelles. In this example a first layer of a cationic copolymer PDEA(26)-b-PDMA(74 non quaternized units and + 22 quaternized units) was deposited before a second layer of an anionic copolymer PDPA(90)-b-PMAA(50); PDEA meaning poly(N,N'-diethylaminoethyl methacrylate), PDMA meaning poly(N,N'-dimethylaminoethyl methacrylate), PDPA meaning poly(diisopropylaminoethyl methacrylate) and PMAA meaning polymethacrylic acid. Figure 3.3 shows that the coating covers all crystal faces, corners and edges.

The presence of micelles all over the surface of the TMX crystals is clearly illustrated in Figure 3.3a and comparison of Figure 3.3b and 3.1b illustrates the crystal surface has been significantly modified by surface coating.

### Zeta potential measurements of the coated TMX particles

The deposition of sequential copolymer layers on the surface of the TMX crystals can be demonstrated by Zeta potential measurements as each layer has a different charge, (PDEA(26)-b-PDMA(74+22) has a positive charge and PDPA(90)-b-PMAA(50) a negative. Using Zeta potential measurements it is possible to track the deposition of at least 5 alternatively charged layers, as shown in Figure 3.4.

The coating procedure, either using a homopolymer or a copolymer layer as an adhesion promoting pre-treatment for the subsequent deposition of a second copolymer layer, was carried out on TMX and tetraacetylethylene diamine (TAED). In addition, several types of copolymers were involved in this coating procedure demonstrating that this procedure is flexible and easily adaptable. Table 3.1 summarises a range of systems prepared using the coating procedure previously outlined. Figures 3.5-3.7 demonstrate the deposition of micelles onto all faces of TMX and TAED.

**Table 3.1**

| Actives | **1st**- layer | 2nd layer | Figure No |
|---|---|---|---|
| | **Cationic** | **Anionic** | |
| TMX | --------- | --------- | 3.1 |
| TMX | PDADMAC | P(BuMA(15)-b-MAA(120)) | 3.2 |
| TMX | PDADMAC | P(TFEMA(20)-b-MAA (140)) | 3.5 |
| TMX | PDEA(26)-b-PDMA(74+22) | PDPA(90)-b-PMAA(50) | 3.3 |
| TMX | PBuMA (26)-b-PDMAEMA(100) | P(BuMA(15)-b-MAA(120)) | 3.6 |
| TAED | PDADMAC | P(BuMA(15)-b-MAA(120)) | 3.7 |

TMX: Thiamethoxam; TAED: tetraacetylethylene diamine; PBuMA: polybutyl methacrylate; PDAMAC: poly(diallyldimethylammonium chloride); PDEA: poly(N,N-diethylaminoethyl methacrylate); PDMA: poly(N,N-dimethylaminoethyl methacrylate);
PDPA: poly(N,N'-diisopropylaminoethyl methacrylate); PMAA: polymethacrylic acid;
PTFEMA: poly(trifluoroethyl methacrylate).

Table 3.1: Description of crystals coated according to the procedure described in the present invention.

### Example 4: Crosslinking and alteration of dissolution profiles

Crosslinking is described as the physical and/or chemical interaction between chains of the AB diblock copolymer. The crosslinking can take place either in the core of the micelles, in the corona of the micelles and/or between the coronas of two contiguous micelles.

In this example, crosslinking of copolymer micelles is used to decrease the solubility of a coated crystalline material in water. Micelles comprising AB di-block copolymers were deposited on the surface of crystals of a crystalline material (for example a pharmaceutical or an agrochemical) in aqueous and oil based liquid media. Addition of linear and cyclic diamine molecules to this system led to the modification of the topology of the micellar coating. This also resulted in a decrease in the release rate of the crystalline material in water.

### A) Example of crosslinking in water based system.

TMX was coated with Poly(BuMA₅₈-b-MAA₅₄) using the protocol described in Example 3a. Following coating the sample was crosslinked following the procedure described below.
1. A diamine compound (see Table 4.1 for mass and molar ratio compared to MAA functions in the copolymer) was added to the solution (1g coated TMX in 10ml TMX saturated stock solution) and tumbled for 48 hours.
2. The mixture was then centrifuged for 2 minutes at 2000rpm and approximately 8ml of the supernatant liquid was removed. The same quantity of TMX saturated stock solution was added, and the mixture tumbled again for 30 minutes.
3. The mixture was then centrifuged for 2 minutes at 2000rpm and 8ml the supernatant liquid was removed, 8ml of TMX saturated stock solution was added.
4. The sample was dried under vacuum at 50°C for 8 hours thus removing all solvents.

A visual release test (i.e. observation of the speed and extent of dissolution) was performed on the coated particles before and after crosslinking. It was found that the crosslinked samples dissolved at a slower rate compared to the uncrosslinked samples.

Crosslinked and uncrosslinked samples were weighed before and after 8 hours in water in order to measure the percentage weight loss. Results are shown in Table 4.1. They confirm the visual release rate observation: TMX samples coated with crosslinked copolymers show less weight loss, in other words less dissolution, than samples coated with un-crosslinked copolymer.

**Table 4.1**

| Cross-linker | Molar ratio of carboxylic acid functions compared to amine functions | Mass of crosslinker used (g) | % of weight loss |
|---|---|---|---|
| Control (coated but not crosslinked) | - | 0.0000 | 84 |
| Methylene bis(cyclohexylamine) | 1 : 1.0 | 0.0715 | 54 |
| Methylene bis(cyclohexylamine) | 1 : 2.1 | 0.1454 | 35 |
| Methylene bis(cyclohexylamine) | 1 : 3.1 | 0.2170 | 38 |
| Hexamethylene diamine | 1 : 2 | 0.0348 | 41 |
| Hexamethylene diamine | 1 : 2.4 | 0.0708 | 36 |
| Hexamethylene diamine | 1 : 3.6 | 0.1057 | 34 |

### Table 4.1: Percentages weight loss of the coated TMX particles

To perform release rate analysis, 45-55mg of each sample was accurately weighted into a 60ml powder jar and 50ml of dispersant solution (0.1%w/w Aerosol OTB, 0.5%w/w Morwet D425 in DI water) added at time zero. The samples were then placed on a roller moving at 20rpm. A time point measurement of TMX in solution was made by extracting 3ml of solution and passing it through a 0.45µm syringe filter. The filtrate was then analysed by HPLC to determine the concentration of TMX. The analysis was carried out by HPLC using an Agilent 1100 (equipped with an auto-injector), a 50 X 3.0 MM ACE 3µM C18 COLUMN FROM ACE, PART NUMBER ACE-111-0503 and mobile phases of (A) Acetonitrile + 0.1% Formic acid and (B) ASTM II Water + 0.1% Formic acid. Analysis was carried out with an injection load of 5µl and column temperature of 40°C. Data were collected at a range of time points.

Total TMX content of the samples was determined by weighing 30-50mg of each dry powder accurately weighted into an aluminium weighting boat. The weighing boat was then placed in a volumetric flask and 50ml acetonitrile added. The flask was gently swirled until a colourless solution was formed. This solution was analysed using the HPLC conditions described previously.

Table 4.2 shows the quantity of TMX release after 1, 8 and 24 hours as a percentage of the total TMX concentration as measured by the method described previously.

**Table 4.2**

| time (hours) | No cross linker | crosslinker Hexamethylene diamine 1:1.5 | crosslinker Methylene bis(cyclohexylamine) 1:1.5 |
|---|---|---|---|
| 1 | 100 | 18 | 29 |
| 4 | 100 | 28 | 44 |
| 24 | 100 | 34 | 81 |

Table 4.2: Quantity of thiamethoxam released over time periods 1, 4, and 24 hours as a percentage of the measured total content. Both crosslinkers were present in a molar ratio of 1:1.5 COOH functions to diamine.

### Example 5: Demonstration of increased polymer deposition using copolymer micelles

The coating procedure described in Example 3 was used to deposit 4 layers of homopolymer electrolyte (PDADMAC / PNaSS / PDADMAC / PNaSS) onto TMX crystals, sample 5.1; and 4 layers of copolymer micelles (P(BuMA₂₆-DMAEMA₉₅ (50% quatemized)/P(BuMA₁₅-MAA₁₂₀)/ (P(BuMA₂₆-DMAEMA₉₅ (50% quaternized)/ P(BuMA₁₅-MAA₁₂₀) onto TMX crystals, sample 5.2.

The samples were then dried in at 40°C in a vacuum oven at (1000mbar below atmospheric pressure) overnight. After this period the liquid was observed to have been removed and dry coated particles of TMX remained. 30-50mg of each dry powder was accurately weighted into an aluminium weighting boat. The weighing boat was then placed in a volumetric flask and 50ml acetonitrile added. The flask was gently swirled until a colourless solution was formed. This solution was analysed to determine the total content of TMX present. The analysis was carried out by HPLC using an Agilent 1100 (equipped with an auto-injector), a 50 X 3.0 MM ACE 3µM C18 COLUMN FROM ACE, PART NUMBER ACE-111-0503 and mobile phases of (A) Acetonitrile + 0.1% Formic acid and (B) ASTM II Water + 0.1% Formic acid. Analysis was carried out with an injection load of 5µl and column temperature of 40°C. Table 5.1 details the mobile phase ratios used during analysis.

**Table 5.1**

| **Time (mins)** | **Mobile phase (A)** | **Mobile phase (B)** | **Flow rate (ml / min)** |
|---|---|---|---|
| 0 | 1 | 99 | 2 |
| 1.8 | 60 | 40 | 2 |
| 2.8 | 95 | 5 | 2 |
| 3.0 | 1 | 99 | 2 |
| 4.0 | 1 | 99 | 2 |

### Table 5.1: Mobile phase ratios for the total content analysis of TMX.

The total content of the TMX samples is shown in Table 5.2

**Table 5.2**

| Sample reference | Total TMX content in the sample %w/w | Total coating content (%w/w) |
|---|---|---|
| (a) Sample 5.1 | 98.55 | 1.45 |
| (b) Sample 5.2 | 91.16 | 8.84 |

### Table 5.2 Total Thiamethoxam (TMX) content of samples coated by 4 layers of (a) homopolymer; and (b) 4 layers of copolymer micelles

Table 5.2 clearly demonstrates that more than 6 times the weight of polymer has been deposited using copolymer micelles rather than homopolymer.

### Example 6: Increasing micelle size by chemical addition

It is well established that micelle size can be increased by adding chemicals which partition into the micelle core. In this example we demonstrate that the micelles from the copolymers in Example 1 can be loaded with chemicals such that a particle size increase is observed. Observation of size alteration in the presence of such chemicals is further demonstration of the presence of micelles.

In a 120 ml screw-top jar, the copolymer (0.1g, 0.5wt%) was dissolved in ethanol (1.6g, 8wt%), under stirring. Water (18.5g, 91.5wt%) was added drop by drop onto this mixture, always under stirring. When the mixture became cloudy, the stirring was stopped. Finally, styrene (40g, twice the mass of the aqueous solution) was poured above the aqueous phase. The two-phase system was left to equilibrate for two days. The lower phase, containing the loaded micelles, was extracted using a pipette and stored for further analysis and/or use.

### (Weight Percentages are given compared to water.)

A Malvern Nano Zetasizer was used to monitor the size of copolymer micelles after the addition of chemicals. In the first instance, styrene was added to copolymer micelles of P(BuMA₁₅-b-MAA₁₂₀). Size distribution measurements shown in Table 6.1 show that the minimum micelle diameter increased from 20 to 30nm.

As expected, a greater increase in micelle size can be obtained by using micelles with a larger hydrophobic core, for example those formed from copolymer P(BuMA₁₂₇-b-MAA₅₁). In this case, size distributions measurements shown in Table 6.1 demonstrate a 29% increase in the average size of the micelles.

**Table 6.1**

| | P(BuMA₁₅-b-MAA₁₂₀) | P(BuMA₁₂₇-b-MAA₅₁) |
|---|---|---|
| Before the loading | 20 - 70nm | 20 - 50nm |
| After the loading | 30 - 70nm | 30 - 60nm |

Table 6.1 Size distribution measurement before and after the loading of copolymer micelles with styrene.

The increase in micelle size in the presence of styrene is further evidence that micelles have been formed.

### Example 7: Coating of Griseofulvin Crystals

Griseofulvin crystals were coated with two layers of copolymer using the protocol as described in section 3a.

The copolymers used were Poly(BuMA₆₀-b-MAA₅₅) and Poly(BuMa₁₅-b-MAA₁₂₀) at 0.4, 1, 2.5 and 5wt%.

A SEM image demonstrating the coating of Griseofulvin with PDADMAC at 0.35wt% and Poly(BuMA₁₅-b-MAA₁₂₀) at 1wt% can be seen in Figure 7.1.

### Example 8 - Targeted delivery of photoprotectants to the crystal interface

The micelle core can be loaded swollen as demonstrated in Example 6. In this example the micelle size was increased by addition of a photoprotectant and the impact on photostability of an agrochemical coated with such loaded micelles was demonstrated. The addition of the photostabiliser can be before or after coating the active ingredient with the copolymer micelles.

To load the micelle prior to coating a Poly(BuMA₁₅-b-MAA₁₂₀) solution in ethanol was prepared (1g polymer, 8g ethanol,) to which 0.5g of 2,6-di-butyl-4-methyl-phenol was added. After complete dissolution water and ammonia solution (35%wt) are added to make a 1%wt copolymer solution at pH 9. This micellar solution was then used to coat 0.86 g of emamectin benzoate particles using the method below.
1. 1g of emamectin benzoate particles was placed in 10ml of deionised water and gently vortexed to disperse the particles.
2. 10ml of the P(BuMA(15)-*b*-MAA(120))/ 2,6-di-butyl-4-methyl-phenol solution described above was added to the emamectin benzoate dispersion.
3. The sample was tumbled for 30 minutes.

Those skilled in the art will recognize that an adhesion promoter can be optionally added. Those skilled in the art will also recognize that the loading of the micelles was not optimized for this example and further swelling of the micelles is possible.

The post loading approach is described below.
1. A 1%wt Poly(BuMA₁₅-b-MAA₁₂₀) micellar solution at pH 9 was prepared in water (1%wt polymer, 8%wt ethanol, 91%wt water/ammonia solution).
2. 0.86g of emamectin benzoate was weighed out and placed in a centrifuge tube and 8.6ml of water was added.
3. The sample was then gently vortexed in order to slowly disperse the active.
4. 8.6ml of 1% micellar solution was added and the sample tumbled for 30 mins.
5. 0.22g of 2,6-di-butyl-4-methyl-phenol and 1.72g of lignin sulphonate [Polyfon™ H] were then added and the dispersion was tumbled until homogenous (in this example the sample was tumbled for 1 hour).

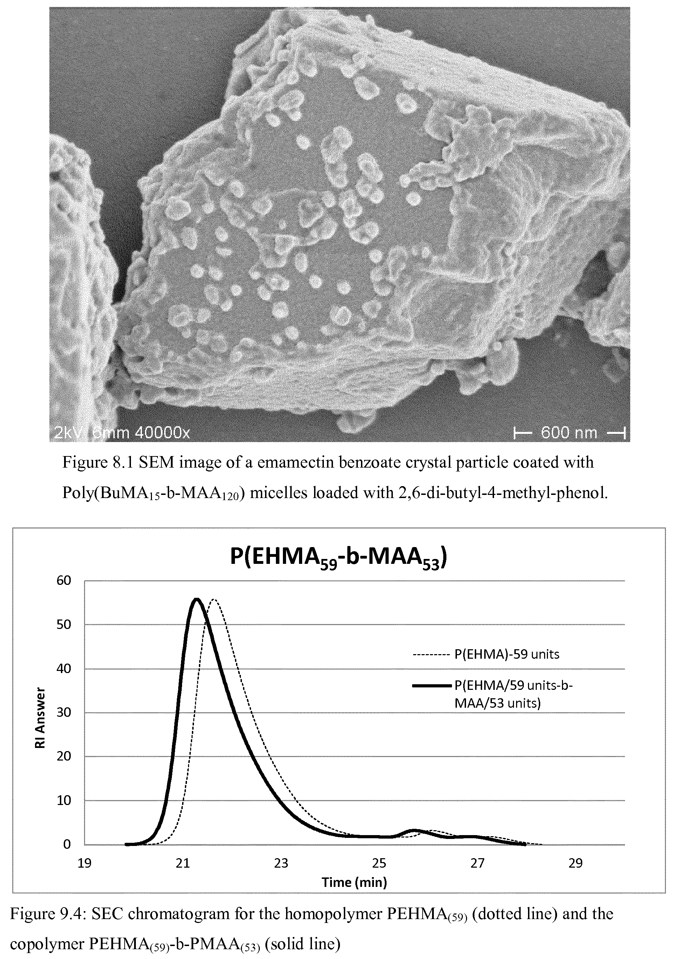

SEM characterisation of the coated sample demonstrates the association of the loaded copolymer micelles with the emamectin benzoate crystal particles, Figure 8.1

The photostability of the coated samples was assessed by irradiating samples and measuring the remaining concentration of emamectin benzoate, by gathering data for a number of time points the half-life for emamectin benzoate under irradiation can be determined.

50ppm dilutions of the emamectin benzoate dispersions were prepared in ultra-pure water. 8 x 2µl drops of these dilutions were applied to glass microscope slides and irradiated at 750W/m² with samples taken after 0, 1h, 3h, 6h, 17h and 25hour irradiation. Deposits removed from slides using 40/50/10 MeCN/0.1% H3PO4/THF solvent and analysed by reverse-phase LC with MS detection. Standards were prepared as follows:
- 8 x 2µl drops of application solution were added directly into liquid chromatogrpahy vials and immediately the solvent was added before storing at 4°C until analysis.
- Time zero samples were prepared by dispensing 8 x 2µl drops of application solution onto a glass microscope slide, allowing the solvent to evaporate and immediately removing the deposit by immersion into the wash-off solvent.

**Table 8.1 demonstrates the impact of photoprotectants in the micellar core on the half-life of emamectin benzoate.**

| description | Emamectin Benzoate concentration (%w/w) | Poly(BuMA_{1 5}-b-MAA₁₂₀) (%w/w) | 2,6-di-butyl-4-methyl-phenol (%w/w) | Ratio of lignin sulfonates to Emamectin Benzoate | Emamectin Benzoate sun test half-life (hours) |
|---|---|---|---|---|---|
| uncoated Emamection Benzoate | 4.8 | 2.4 | - | - | 8 |
| Emamectin Benzoate coated with copolymer | 4.8 | 2.4 | - | - | 6 |
| Emamectin Benzoate coated with copolymer and lignin sulfonates | 4.4 | 2.2 | - | 2:1 | 8 |
| Emamectin Benzoate coated with copolymer + post-loaded (BHT) + lignin sulfonates | 4.4 | 2.2 | 1.1 | 2:1 | 14 |

Table 8.1 The effect of copolymer micelles and photoprotectants on the half-life of Emamectin Benzoate

### Example 9: Preparation of Polymers and Block Copolymers for micelle formation in apolar liquids

The surface treatment of the present invention is hydrophobic. The copolymers described in this example are AB block copolymers comprising a substantially hydrophobic block A, and a substantially hydrophobic or hydrophilic block B which has a different affinity for or solubility parameter within the liquid media where the copolymers are dispersed compared to block A, such that micelles form in the liquid medium.

Block A can comprise of one or more monomers, for example; styrene (S) and styrene derivatives, methacrylate and derivatives such as 2-ethyl hexyl methacrylate (EHMA), lauryl methacrylate (LMA), octadecyl methacrylate (ODMA), glycidyl methacrylate (GMA) and propylene oxide (PO). Those skilled in the art will appreciate the synthesis described in this example is not limited to the monomers listed here.

In the current example, the hydrophobic or hydrophilic block B was composed of methacrylic acid (MAA), 2-hydroxyethyl methacrylate (HEMA) or 2-ethyl hexyl methacrylate but those skilled in the art will understand that other monomers leading to a hydrophilic block can also be used.

The copolymers used herein were produced by Reversible Addition-Fragmentation Chain Transfer (RAFT) according to the protocol described in the patent applications WO08071957 and WO10038046 or by nitroxide mediated polymerisation (NMP) according to the protocol described in WO2007/057620A1. Therefore the block copolymers may be prepared by means of controlled living polymerisation techniques, such as group transfer polymerisation (GTP), atomic transfer radical polymerisation (ATRP), and activated regenerated by electron transfer (ARGET) or activated generated by electron transfer (AGET) that can synthesize well-defined homopolymers and block copolymers.

Examples of the composition of new prepared copolymers are given in Table 9.2.

### A) Use of RAFT to synthesise copolymers

In this example, in addition to structures described in WO08071957 and WO10038046, new copolymers structures were produced by RAFT polymerization using the RAFT agent, 2-cyanoisopropyl dithiobenzoate (CPDB). Whilst the current example prepares the block copolymer CPDB, those skilled in the art will appreciate that other RAFT agents may be used.

### RAFT synthesis of Poly(EHMA-block-MAA) copolymers : P(EHMA-b-MAA)

A series of poly[EHMAₓ-*b*-MAA_{y}] copolymers were prepared by RAFT polymerization using CPDB as chain transfer agent, azobisisobutyronitrile (AIBN) as initiator and propan-2-ol (IPA) as a solvent. The synthesis was a two step process: First, the hydrophobic block (EHMA) was synthesised, then the synthesis of the hydrophilic block (MAA) was initiated from the PEHMA homopolymer.

### a) Synthesis of the block A: PEHMA.

EHMA (15g, 75.7mmol, 60eq), CPDB (0.31g, 1.26mmol, 1eq), AIBN (0.10g, 0.63mmol, 0.5eq) and IPA (solvent, 6.82g, 114mmol) were added in a two necked flask containing a magnetic stirrer equipped with a cooling column. The mixture was degassed by nitrogen bubbling and heated at 90°C in a thermostatically controlled oil bath under a nitrogen atmosphere. The reaction was left under stirring for a minimum of 2hours 30minutes (in this example 3h15min). A sample of the crude mixture was withdrawn and analysed by size exclusion chromatography (SEC - See Figure 9.4), and by Proton Nuclear Magentic Resonance (¹H NMR). A conversion of 98% was determined by ¹H NMR in CDCl₃, hence the resultant product was P(EHMA)ₓ homopolymer where x = 59.

### b) Synthesis of block B from block A

30 minutes before the end of the first synthesis, MAA (6.54g, 76.0mmol, 60 q), AIBN (0.10g, 0.64mmol, 0.5eq) and IPA (solvent, 45.39g, 757mmol) were added in another flask containing a magnetic stirrer. The mixture was degassed by nitrogen bubbling.

At the end of the first synthesis (in the current example 3h15min), the thermostatically controlled oil bath was removed to stop polymerisation. The mixture containing the second monomer was then transferred into the initial two necked flask via a cannula. This flask was heated again at 85°C in the thermostatically controlled oil bath (equipped with a cooling column) under nitrogen atmosphere to achieve the preparation of the second block of copolymer. After a minimum of 2h30min (in this example 2h35min), a sample of the crude mixture was withdrawn and analysed by ¹H NMR and SEC (Figure 9.4).

A conversion of 88% was measured by ¹H NMR in DMSO. The resultant product was determined as P(EHMAₓ-*b*-MAA_{y}) copolymer where x = 59 and y = 53.

Other P(EHMAₓ-*b*-MAA_{y}) polymers were prepared with x=68 and y=25 and with x=33 and y=21. The generic structure of the corresponding P(EHMAₓ-*b*-MAA_{y}) copolymers is given below.

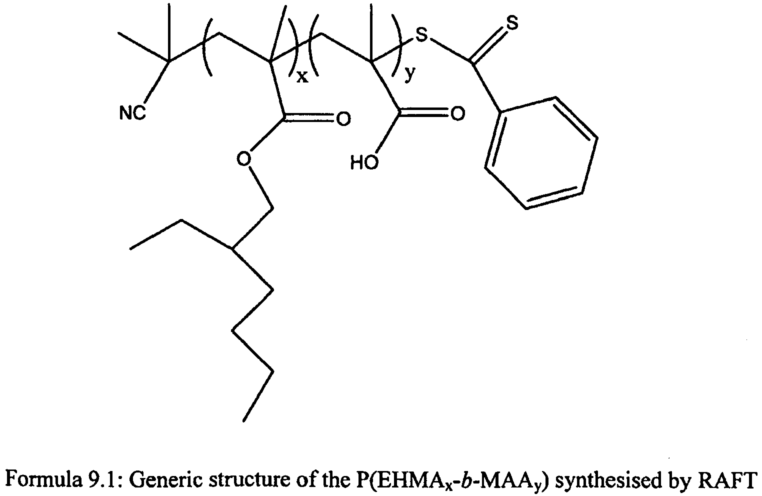

The P(EHMAₓ-*b*-MAA_{y}) copolymers can also be prepared by NMP, ATRP, GTP and indirect anionic polymerization.

### Preparation of other copolymers by RAFT synthesis.

Various block copolymers were synthesised. Block A was obtained from various methacrylated based monomers such as EHMA, LMA, ODMA and TFEMA. Block B was composed of hydrophilic units such as MAA and HEMA, or hydrophobic monomers such as EHMA. In this case, toluene was the solvent used for the synthesis instead of isopropanol.

The method described above for the synthesis of P(EHMAₓ-b-MAA_{y}) was used, which led for example to the successful synthesis of P(LMAₓ-b-EHMA_{y}) and P(ODMAₓ-b-MAA_{y}). The conversion rates, block sizes and reaction time are given in Table 9.2.
For the synthesis of (PEHMA₅₁-r-PGMA₂₂)-b-PMAA₄₇ the following protocol was used:

### a) Synthesis of the block A: PGMA and EHMA

GMA (3.29g, 23.2mmol, 25.6eq), EHMA (11.01g, 55.6mmol, 61.3eq), CPDB (0.22g, 1mmol, eq), AIBN (0.08g, 0.5mmol, 0.5eq) and IPA (solvent, 24.32g, 407mmol) were added in a two necked flask containing a magnetic stirrer equipped with a cooling column. The mixture was degassed by nitrogen bubbling and heated at 82°C for 5 hours in a thermostatically controlled oil bath under a nitrogen atmosphere and then reduced to 70°C for another 16 hours. A sample was removed for NMR analysis. A conversion of 93% GMA and 89% EHMA was measured by ¹H NMR in CDCl₃.

### b) Synthesis of block B from block A

30 minutes before the end of the first synthesis, MAA (4.7882g, 55.6mmol, 56.4eq), AIBN (0.0771g, 0.5mmol, 0.5eq) and IPA (solvent, 24.5034g, 408.7mmol) were added in another flask containing a magnetic stirrer. The mixture was degassed by nitrogen bubbling.

At the end of the first synthesis, the thermostatically controlled oil bath was removed to stop polymerisation. The mixture containing the second monomer was then transferred into the initial two necked flask via a cannula. This flask was heated again at 82°C for 4 hours in the thermostatically controlled oil bath (equipped with a cooling column) under nitrogen atmosphere and then reduced to 70°C for 16 hours to achieve the preparation of the second block of copolymer. Polymers were precipitated out in diethyl ether and dried in a vacuum oven at 40°C.

A conversion of 82% for the MAA was measured by ¹H NMR in DMSO. The resultant product was determined as (PEHMAₓ-r-PGMA_{y})-b-PMAA_{z} copolymer where x = 51, y = 22 and z = 47.

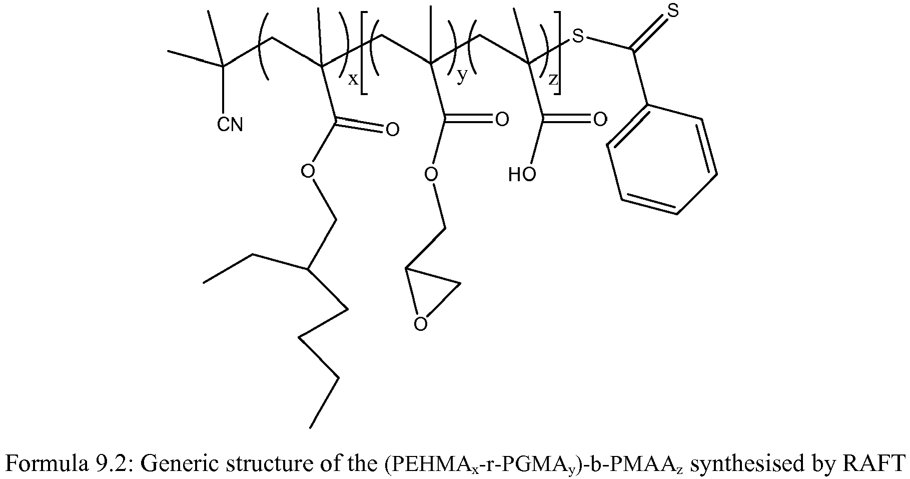

### B) Use of NMP to synthesise copolymers

In this example, according to the protocol described in the patent WO2007/057620A1, new copolymers structures were produced by NMP polymerization using the NMP agent Blocbuilder®. Whilst the current example prepares the block copolymer using Blocbuilder®, those skilled in the art will appreciate that other NMP agents may be used.

### NMP synthesis of PSₓ-b-(HEMA_{y}-r-PS_{z})

In the first step, the following conditions were used for the synthesis of PS with a targeted polymerisation degree of 55. Styrene (15.00g, 0.14mol) and Blocbuilder^{®} (1.00g, 2.62mmol) were added to a 100ml round bottom flask equipped with a magnetic stirrer. The reaction flask was degassed by nitrogen bubbling for 20 minutes and then heated at 90°C in a thermostatically controlled oil bath under a nitrogen atmosphere. After 78hr40 min of polymerization, a sample was withdrawn and analysed by ¹H NMR (CDCl₃). A conversion of 76.9% was determined by ¹H NMR in CDCl₃, hence the resultant product was PSₓ homopolymer where x = 42.

At the end of this step 15g chloroform was added to solubilise PS. The reactive mixture was precipitated drop by drop in 300ml cold methanol and then filtered on paper. The product was dried down in a vacuum oven.

In a second step, newly synthesised PS (1.00g, 0.23mmol), styrene (0.24g, 2.32mmol), HEMA (2.95g, 22.7mmol) and dimethylformamide (DMF, 4.02g, 0.55mmol) were added to a 50ml round bottom flask equipped with a magnetic stirrer. PS was solubilised in DMF by using a sonic bath (20min). The reaction flask was degassed by nitrogen bubbling for 20 minutes and then heated at 90°C in a thermostatically controlled oil bath under a nitrogen atmosphere. After 18 hours of polymerization, a sample was withdrawn and analysed by ¹H NMR (DMSO). A conversion of 90.0% for HEMA and 8.0% for styrene was determined by ¹H NMR in DMSO, hence the resultant product was PSₓ-b-(HEMA_{y}-r-PS_{z}) diblock copolymer, where x = 42, y = 90 and z = 8.

At the end of this step, 7mL DMF was added to solubilise the copolymer. The reactive mixture was precipitated drop by drop in 300ml cold ether and then filtered on paper. The product was dried down in a vacuum oven.

Other PSₓ-b-(HEMA_{y}-r-PS_{z}) were prepared with x=86, y=57 and z = 0 and with x=74, y=30 and z=10. The generic structure of the corresponding PSₓ-b-(HEMA_{y}-r-PS_{z}) copolymers is given below.

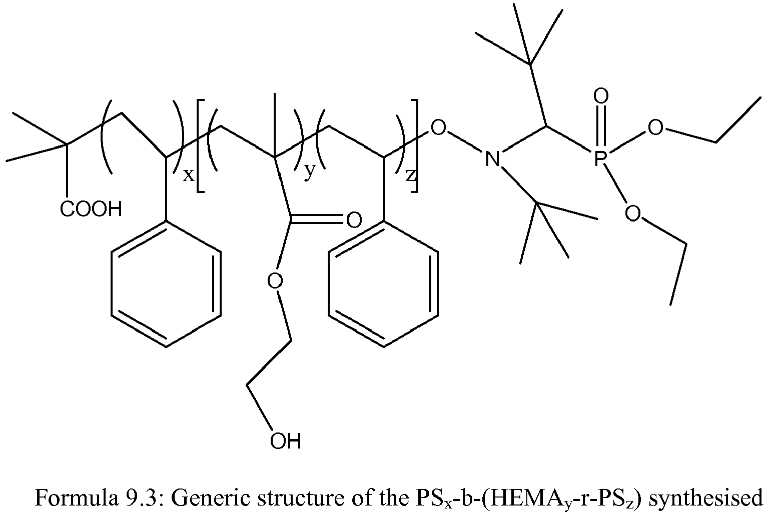

### C) Characterisation

SEC was used to determine the number-average molar mass (Mₙ) and thus demonstrate the increase of molar mass due to the addition of the second block during the polymerisation. SEC was also used to determine the polydispersity index (PDI= M_{w}/Mₙ, where M_{w} is the weight-average molar mass) of the polymers and copolymers, a low PDI being necessary to achieve regular micelles.

The samples were injected in the SEC equipment (2 PL gel 5 Micron Mixed-c columns) and analysis was performed as described below
- The eluent was composed of tetrahydrofuran (THF) for P(EHMAₓ-*b*-MAA_{y}) copolymers and DMF for PSₓ-b-(HEMA_{y}-r-PS_{z}) copolymers (elution flow rate: 1ml/min, run time: 30 min).
- The calculation (for data analysis) was made with a calibration curve based on poly(methyl methacrylate).
- Before injecting the polymer samples containing methacrylic acid units, a methylation reaction was performed to convert the acid groups into methyl esters, using trimethylsilyldiazomethane as the methylating agent, in order to solubilise the polymers in THF to perform the analysis.
- The samples (20mg) were dissolved in the eluent and then filtered with a 0.2µm PTFE filter into the SEC vials.

An example of SEC chromatogram is given in Figure 9.4. The SEC chromatogram of the first block of P(EHMA) and the chromatogram of the copolymer P(EHMA-*b*-MAA) are represented. The observed shift of the chromatogram is consistent with an extension of chains between both steps.

**Table 9.1**

| Copolymer | PDI - block 1 | PDI - block 2 |
|---|---|---|
| P(EHMA₅₉-b-MAA₅₃) | 1.68 | 1.85 |
| P(S₄₂-b-[HEMA₉₀-r-S₈]) | 1.31 | 2.03 |
| P(S₈₆-b-HEMA₅₇) | 1.65 | 1.58 |
| P(S₇₄-b-[HEMA₃₀-r-S₁₀]) | 1.43 | 1.84 |
| P(LMA₃₃-b-HEMA₁₀₆) | 1.23 | 1.62 |

Table 9.1: Indication of PDI obtained by SEC for some copolymers described in Table 9.2
¹H NMR was used to determine the conversion of each polymerisation and the degree of polymerisation (in number: DPₙ) calculated accordingly for each block. ¹H NMR was performed with a 500MHz apparatus (Bruker), in CDCl₃ for the homopolymer, and in DMSO for the copolymer.

**Table 9.2**

| | Block 1 | | | Block 2 | | |
|---|---|---|---|---|---|---|
| Copolymer | DPn th (units) | Conv. / time | DPn exp x = | DPn th (units) | Conv. / time | DPn exp y = |
| P(EHMAₓ-b-MAA_{y}) ⁽ⁱ⁾ | 60 | 98%, 3h15 | 59 | 60 | 88%, 2h35 | 53 |
| P(Sₓ-b-[HEMA_{y}-r-S_{z]})⁽ⁱⁱ⁾ | 80 | 92%, 70h30 | 74 | y: 50 z: 10 | 60%, 24h | y = 30 z = 10 |
| P(Sₓ-b-HEMA_{y}) ⁽ⁱⁱ⁾ | 55 | 76.9%, 78h40 | 42 | y: 100 z: 10 | 90%, 18h | y = 90 z = 8 |
| P(LMAₓ-b-EHMA_{y}) ⁽ⁱⁱⁱ⁾ | 35 | 95.3%, 1h30 | 33 | 106 | 100%, 2h05 | 106 |
| P(EHMAₓ-b-ODMA_{y}) ⁽ⁱⁱⁱ⁾ | 42 | 95.2 % 1h15 | 40 | 70 | 100 %, 19h00 | 70 |

Table 9.2: Synthesis and composition data according to ¹H NMR; EHMA: 2-ethyl hexyl methacrylate; HEMA: 2-hydroxyethyl methacrylate ; MAA: methacrylic acid; S: styrene; LMA: lauryl methacrylate; ODMA: octadecyl methacrylate; Conv.: conversion given in %; DPn th: degree of polymerisation targeted; DPn exp: degree of polymerisation calculated;
i) Synthesis performed using RAFT in IPA
ii) Synthesis performed using NMP in DMF
iii) Synthesis performed using RAFT in toluene

### Example 10: Demonstration of micelle formation in apolar liquid media

Micellar aggregates can be formed from the copolymers of Example 9. Size distribution measurements using a Malvern Nano Zetasizer were performed on solutions of in apolar solvents such as dodecane, hexane, Exxsol D140, Solvesso 200ND and Isopar V.
1. To demonstrate the formation of micelles in apolar solvent, a solution (10 to 20ml) of copolymer was prepared by dissolving the copolymer powder in -THF (Sigma-Aldrich) (1wt%).
2. When the polymer had dissolved, a second solvent as indicated in Table 10.1 was added drop-by-drop until it reached a large enough quantity that it became the continuous phase. For size distribution measurements, this was when the concentration of copolymer reached ∼0.01 wt%.
3. To ensure that equilibrium was reached the mixture was gently agitated for over one hour (mixing with a magnetic stirrer set on low).

To ensure an accurate measurement by the Malvern Nano Zetasizer, concentrations of the copolymer solution were varied so the sample was in the optimum detection range of the instrument for the polymer being examined. The size distribution measurements shown in Table 10.1 shows that the copolymers form micelles, since the minimum diameter measured was 20nm and if copolymers were present as unimers, the diameter would have been less than 5nm. In all cases a clear solution was formed following stage 1. The results in Table 10.1 demonstrate that in each case micelles were formed following stage 3.

Examples of hydrophobic copolymer micelles solutions which were prepared according to the general procedure are described in Table 10.1.

**Table 10.1: Micelle size measurements of copolymer P(Ethyl Hexyl MA(29)-b-MAA(48)) in apolar liquid media. Measurements collected using a Malvern Nano Zetasizer.**

| size distribution following stage 3 (nm) | | | | |
|---|---|---|---|---|
| Dodecane | Hexane | Exxsol D140 | Solvesso 200ND | Isopar V |
| 230 - 240 | 100 - 110 | 95 - 105 | 20-80 | 25 - 70 |

### Example 11 - Coating of a crystalline particle

A copolymer solution was prepared by dissolving the copolymer in a good solvent (toluene / THF) under gentle agitation. Once a homogeneous solution was obtained a second solvent (for example hexane / Isopar V) was added to the mixture using drop-by-drop addition. The final concentration of the copolymer in solution was 0.4wt%. The second solvent was selected to be a poor solvent or non-solvent for one of the blocks and a good solvent for the other block. The mixture was gently stirred and left for more than 2 hours in order to allow the copolymers to equilibrate into micelles. When the micelle system had reached equilibrium, 1g of TMX air milled crystals was added to the mixture. The sample was then allowed to tumble for at least 2 hours in order to ensure complete mixing and thus allow time for the micelles to coat the individual TMX crystals. Table 11.1 shows possible but not limiting combinations of copolymers and organic solvents which form micelles and can be used to coat crystals.

**Table 11.1**

| **Mixtures** | **Copolymer actives** | **Step 1: Addition of the good solvent** | | **Step 2: Addition of the second solvent** | |
|---|---|---|---|---|---|
| | | **Name** | **Volume (g)** | **Name** | **Volume (g)** |
| 1 | **PEHMA**(**59**)-**b-PMAA**(**53**) | Toluene | 0.96 | Hexane | 9 |
| 2 | **PEHMA**(**59**)-**b-PMAA**(**53**) | Toluene | 0.96 | Isopar V | 9 |
| 3 | **PEHMA**(**59**)-**b-PMAA**(**53**) | THF | 0.96 | Hexane | 9 |
| 4 | **PEHMA**(**59**)-**b-PMAA**(**53**) | THF | 0.96 | Isopar V | 9 |
| 5 | **PEHMA**(**29**)-**b-PMAA**(**48**) | Toluene | 0.96 | Hexane | 9 |
| 6 | **PEHMA**(**29**)-**b-PMAA**(**48**) | Toluene | 0.96 | Isopar V | 9 |
| 7 | **PEHMA**(**29**)-**b-PMAA**(**48**) | THF | 0.96 | Hexane | 9 |
| 8 | **PEHMA**(**29**)-**b-PMAA**(**48**) | THF | 0.96 | Isopar V | 9 |

| | | | | | |
|---|---|---|---|---|---|
| PMAA: polymethacrylic acid; PEHMA polyethylhexylmethacrylate | | | | | |

### Table 11.1: Composition of copolymers solutions in apolar hexane and in Isopar V

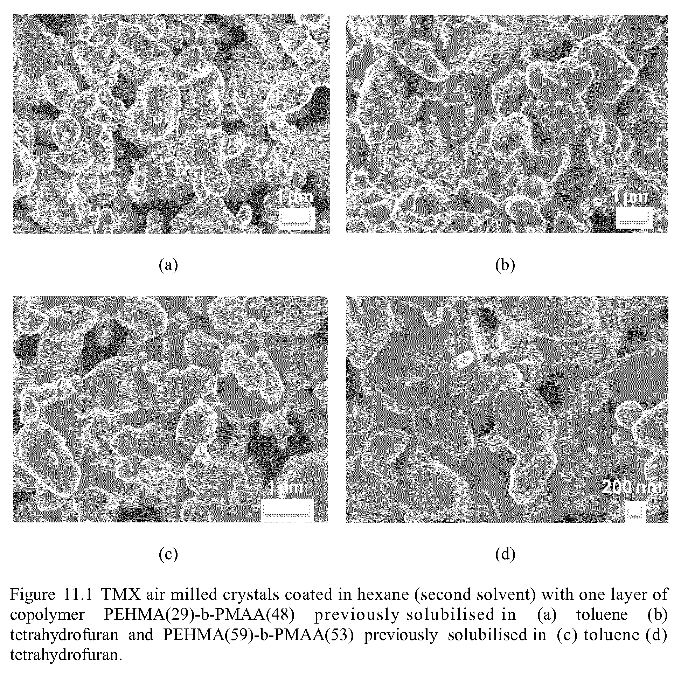

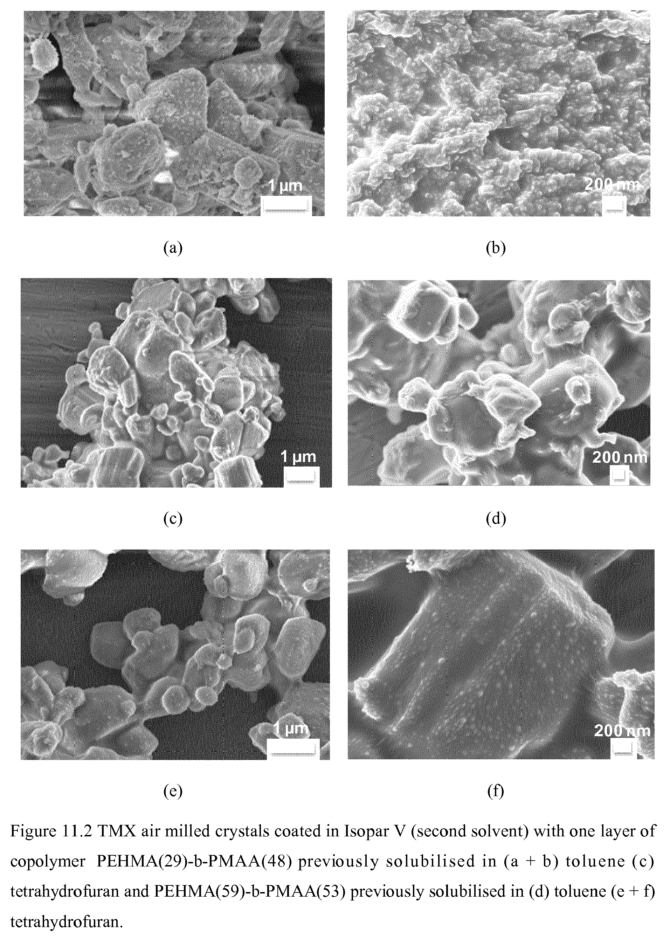

The copolymer solutions in Table 11.1 were used to coat TMX particles using the methodology outlined previously. Figures 11.1 and 11.2 demonstrate that micelles have been deposited from the organic solutions.

Figures 11.1 and 11.2 clearly illustrate the deposition of micelles from a range of organic solvents on all crystal faces, including corners and edges of TMX.

### Example 12: Crosslinking of copolymer micelles

Crosslinking is described as the physical and/or chemical interaction between chains of the AB diblock copolymer. The crosslinking can take place either in the core of the micelles, in the corona of the micelles and/or between the coronas of two contiguous micelles.

In this example, crosslinking of copolymer micelles was used to decrease the solubility of a coated crystalline material in water. Micelles comprising AB di-block copolymers were deposited on the surface of crystals of a crystalline material (for example a pharmaceutical or an agrochemical) in oil based liquid media. Addition of either linear or cyclic diamine molecules to this system led to the modification of the topology of the micellar coating. This also resulted in a decrease in the release rate of the crystalline material in water compared to crystalline material coated with un-crosslinked copolymer micelles.

### Example of crosslinking in oil based system

TMX was coated by using the same protocol as example 11.

A copolymer solution (10g) was prepared by dissolving (PEHMA₅₁-r-PGMA₂₂)-b-PMAA₄₇ copolymer in a good solvent (THF) under gentle agitation. Once a homogeneous solution was obtained a second solvent (hexane) was added to the mixture using drop-by-drop addition. The final concentration of the copolymer in solution was 0.4wt%. The second solvent was selected to be a poor solvent or non-solvent for one of the blocks and a good solvent for the other block. The mixture was gently stirred and left for 24 hours in order to allow the copolymers to equilibrate into micelles. When the micelle system had reached equilibrium, 1g of TMX air milled crystals was added to the mixture. The sample was then allowed to tumble for 24 hours in order to ensure complete mixing and thus allow time for the micelles to coat the individual TMX crystals.

Crosslinking was then performed.
5. A diamine compound (see Table 12.1 for mass and molar ratio compared to MAA functions in the copolymer) was added to the solution and tumbled for 24 hours.
6. The mixture was then centrifuged for 2 minutes at 2000rpm and approximately 9ml of the supernatant liquid was removed. The same quantity of water based TMX saturated stock solution was added, and the mixture tumbled again for 30 minutes.
7. The mixture was then centrifuged for 2 minutes at 2000rpm and 9ml the supernatant liquid was removed.
8. The sample was then dried under vacuum at 50°C for 8 hours thus removing all remaining solvents.

**Table 12.1**

| Cross-linker | Molar ratio of (carboxylic acid + epoxy) functions compared to amine | Mass of crosslinker used (g) |
|---|---|---|
| Control (coated but not crosslinked) | - | |
| Hexamethylene diamine (0.4 wt%) | 1 : 3.8 | 0.0348 |
| Hexamethylene diamine (5 wt%) | 1 : 1.1 | 0.1566 |

### Table 12.1 Percentages weight loss of the coated TMX particles

To perform release rate analysis 45-55mg of each sample was accurately weighted into a 60ml powder jar and 50ml of dispersant solution (0.1%w/w Aerosol OTB, 0.5%w/w Morwet D425 in deionised water) added at time zero. The samples were then placed on a roller moving at 20rpm. A time point measurement of TMX in solution was made by extracting 3mls of solution and passing it through a 0.45µm filter. The filtrate was then analysed by HPLC to determine the concentration of TMX. The analysis was carried out by High-performance liquid chromatography (HPLC) using an Agilent 1100 (equipped with an auto-injector), a 50 X 3.0 MM ACE 3µM C18 COLUMN FROM ACE, PART NUMBER ACE-111-0503 and mobile phases of (A) Acetonitrile + 0.1% Formic acid and (B) ASTM II Water + 0.1% Formic acid. Analysis was carried out with an injection load of 5µl and column temperature of 40°C. Data was collected at a range of time points

Total TMX content of the samples was determined by weighing 30-50mg of each dry powder accurately weighted into an aluminium weighting boat. The weighing boat was then placed in a volumetric flask and 50 ml acetonitrile added. The flask was gentle swirled until a colourless solution was formed. This solution was analysed using the HPLC conditions described previously.

Table 12.2 shows the quantity of TMX released after 1 and 4 hours as a percentage of the total TMX concentration as measured by the method described previously.

**Table 12.2**

| time (hours) | Poly(EHMA₅₁-r-GMA₂₂-b-MAA₄₇) no crosslinker | Poly(EHMA₅₁-r-GMA₂₂-b-MAA₄₇) crosslinked (1:1.5 GMA to HMDA ratio) | Poly(EHMA₅₁-r-GMA₁₃-b-MAA₂₁) (1:1.5 GMA to HMDA ratio) |
|---|---|---|---|
| 1 | 79 | 36 | 59 |
| 4 | 84 | 70 | 70 |

### Example 13: Use of micelles from an apolar solution to coat -actives used in the field of laundry.

Sodium percarbonate crystals were coated by adding a copolymer micellar solution of Poly(PS₄₂-b-HEMA₆₉) at 0.4 and 5wt% in DMF/Solvesso.

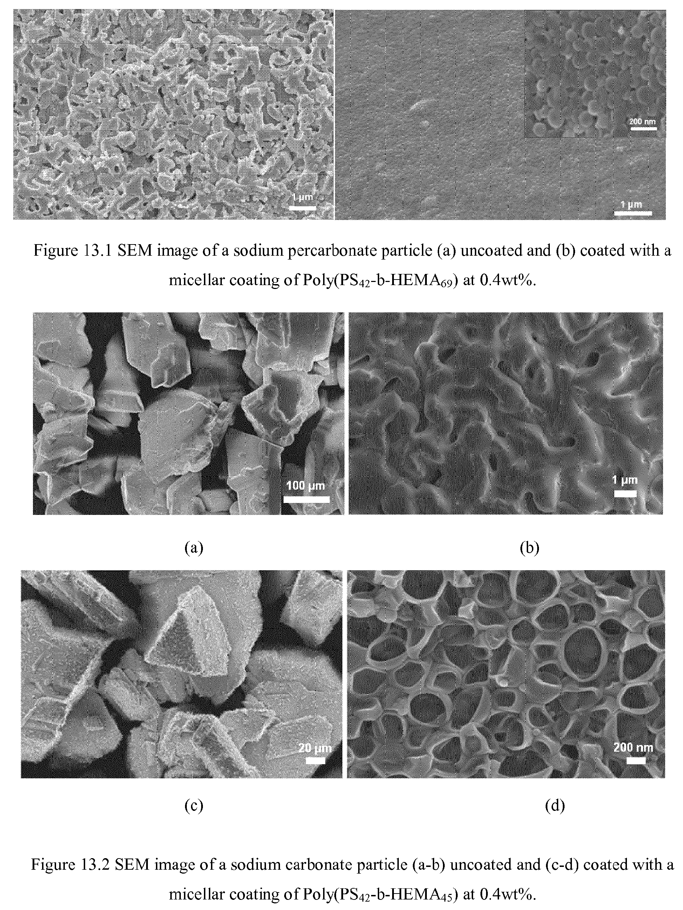

The protocol described in Example 11 was used to coat sodium percarbonate - see Figure 13.1.

The protocol described in Example 11 was used to coat sodium carbonate crystals with micelles of Poly(PS₄₂-b-HEMA₄₅) via a DMF/Solvesso™ 200 liquid medium - see Figure 13.2.

### Example 14: Use of oil-based micelles to coat - actives used in the field of taste masking

Bitrex was chosen as it is the bitterest chemical known to man, and has similar physical and chemical characteristics to many pharmaceuticals.

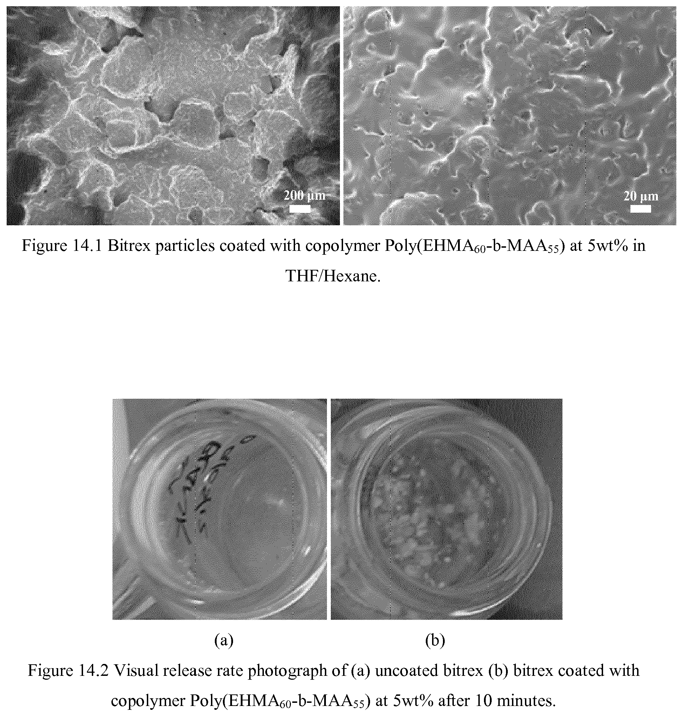

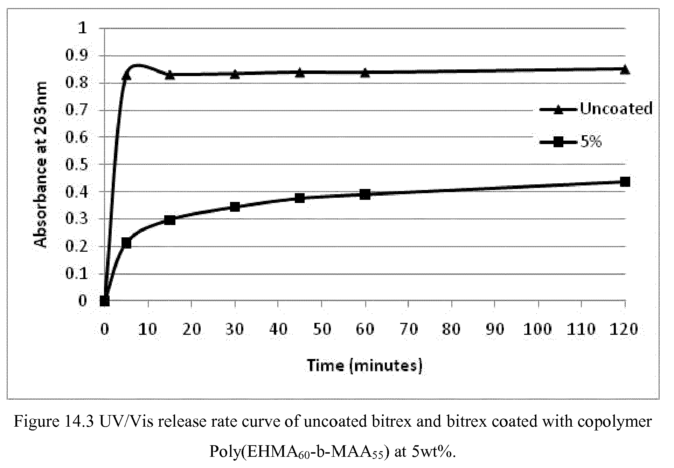

The protocol described in Example 11 was used to coat Bitrex, denatonium benzoate, with copolymer micelles of Poly(EHMA₆₀-b-MAA₅₅) via DMF/Solvesso™ 200 liquid medium - see Figure 14.1.

### Visual release rate test

Release rate was monitored visually in order to compare the uncoated Bitrex with the 5wt% coated Bitrex particles. 0.4mg of sample was agitated in 10ml of water and observed over 8 hours. After 15 minutes the uncoated Bitrex was fully dissolved but after 8 hours the coated particles were still present - see Figure 14.2.

### UV/Vis release rate measurement

100mg in 40ml water of uncoated Bitrex and 5wt% coated Bitrex was shaken for a period of 10 minutes and sampled at various time intervals. 2ml of the mixture was removed at each time interval for analysis.

Total content of the samples was determined by the accurately weighing 17.5mg of 5wt% coated Bitrex particles was sonicated until the coated particles had fully dissolved in 25ml of water and analysed by UV/Vis. A total content measurement of 57.75% was obtained - see Figure 14.2.

**Table 14.1**

| **Time (minutes)** | **% release of Bitrex** | |
|---|---|---|
| | **Uncoated** | **Coated** |
| 5 | 99.6 | 46.5 |
| 15 | 99.8 | 64.9 |

Table 14.1 % release of micelle coated and uncoated Bitrex relative to the total content as determined by UV/vis measurements.

## Claims

1. An organic crystalline particle coated by at least 10 micelles which themselves comprise an AB block copolymer which comprises (i) a first hydrophobic block A, comprising a polymer selected from the group consisting of a homopolymer of an acrylate or alkylacrylate monomer; a copolymer comprising two or three different monomers selected from acrylate or alkylacrylate monomers; a homopolymer of a styrenic derivative monomer; a copolymer comprising two different monomers selected from styrenic derivative monomers; a homopolymer of an alkene or diene monomer; a copolymer comprising two different monomers selected from alkene and diene monomers; a homopolymer of a heterocyclic monomer; and a random, alternating, gradient or block copolymer comprising monomers selected from acrylate monomers, alkylacrylate monomers, styrenic derivative monomers, alkene monomers and diene monomers; and (ii) either a second hydrophobic block B or a hydrophilic block B having a different affinity than the block A for the liquid medium in which the AB copolymers are dispersed such that micelles are formed.

2. An organic crystalline particle as claimed in claim 1 where the particle is or comprises a biologically active compound.

3. An organic crystalline particle as claimed in claim 2 where the biologically active compound is an agrochemical or pharmaceutical.

4. An organic crystalline particle as claimed in any one of the preceding claims where the largest dimension of the particle is from 5mm to 10nm.

5. An organic crystalline particle as claimed in any one of the preceding claims where the polymer has a molecular weight of from 3000 to 100000Dalton.

6. An organic crystalline particle as claimed in any one of the preceding claims where the micelles have a largest dimension of from 3 to 500nm.

7. An organic crystalline particle as claimed in any one of the preceding claims where the micelles each comprise from 10 to 1000 copolymer molecules.

8. An organic crystalline particle as claimed in any one of the preceding claims where the micelles are crosslinkable.

9. An organic crystalline particle as claimed in any one of the preceding claims where the particle is entirely coated by the micelles.

10. An organic crystalline particle as claimed in any one of the preceding claims where the cores of the micelles contain a chemical.

11. An organic crystalline particle as claimed in claim 10 where the chemical contained in the cores of the micelles is a photoprotectant.

12. An organic crystalline particle as claimed in claim 10 where the chemical contained in the cores of the micelles is a biologically active compound.

13. An organic crystalline particle as claimed in claim 10 where the chemical contained in the cores of the micelles is an adjuvant.

14. A composition comprising a plurality of coated organic crystalline particles where each of those particles is an organic crystalline particle as claimed in any one of the preceding claims.

15. A composition as claimed in claim 14 where the composition is a solid composition.

16. A composition as claimed in claim 14 where the organic crystalline particles are dispersed in a liquid.

17. A composition as claimed in claim 16 where the ratio by weight of the copolymer to the liquid is from 1:100000 to 1:1.

18. A composition as claimed in claim 16 or 17 where the liquid comprises water.

19. A composition as claimed in claim 16 or 17 where the liquid is non-aqueous.

20. A composition as claimed in claim 19 where the micelles are reverse micelles.

21. A process for preparing a coated organic crystalline particle as claimed in any one of claims 1 to 13 comprising the steps of
(a) forming micelles of the copolymer; and
(b) mixing the micelles with an organic crystalline particle.

22. A process as claimed in claim 21 where the micelles are crosslinked before, during or after mixing the micelles with the organic crystalline particle.

23. Use of an organic crystalline particle as claimed in any one of claims 1 to 13 or use of a composition as claimed in any of claims 14 to 20 to combat or control an agricultural pest where the organic crystalline particle is or comprises a biologically active compound which is an agrochemical.

## Patentansprüche

1. Organisches kristallines Partikel, das mit mindestens 10 Mizellen beschichtet ist, die selbst wiederum ein AB-Block-Copolymer umfassen, welches Folgendes umfasst: (i) einen ersten hydrophoben Block A, umfassend ein Polymer, ausgewählt aus der Gruppe bestehend aus einem Homopolymer eines Acrylat- oder Alkylacrylatmonomers; ein Copolymer, umfassend zwei oder drei unterschiedliche Monomere, ausgewählt aus Acrylat- oder Alkylacrylatmonomeren; ein Homopolymer eines Styrolderivatmonomers; ein Copolymer, umfassend zwei unterschiedliche Monomere, ausgewählt aus Styrolderivatmonomeren; ein Homopolymer eines Alken- oder Dienmonomers; ein Copolymer, umfassend zwei unterschiedliche Monomere, ausgewählt aus Alken- und Dienmonomeren; ein Homopolymer eines heterocyclischen Monomers; und ein statistisches, alternierendes, geneigtes oder Block-Copolymer, umfassend Monomere, ausgewählt aus Acyrlatmonomeren, Alkylalcrylatmonomeren, Styrolderivatmonomeren, Alkenmonomeren und Dienmonomeren; und (ii) entweder einen zweiten hydrophoben Block B oder einen hydrophilen Block B mit einer unterschiedlichen Affinität als der Block A für das flüssige Medium, in dem die AB-Copolymere so dispergiert sind, dass Mizellen gebildet werden.

2. Organisches kristallines Partikel nach Anspruch 1, wobei das Partikel ein biologischer Wirkstoff ist oder einen biologischen Wirkstoff umfasst.

3. Organisches kristallines Partikel nach Anspruch 2, wobei es sich bei dem biologischen Wirkstoff um eine Agrarchemikalie oder ein Pharmazeutikum handelt.

4. Organisches kristallines Partikel nach einem der vorhergehenden Ansprüche, wobei die größte Dimension des Partikels 5 mm bis 10 nm beträgt.

5. Organisches kristallines Partikel nach einem der vorhergehenden Ansprüche, wobei das Polymer ein Molekulargewicht von 3000 bis 100 000 Dalton aufweist.

6. Organisches kristallines Partikel nach einem der vorhergehenden Ansprüche, wobei die Mizellen eine maximale Dimension von 3 bis 500 nm aufweisen.

7. Organisches kristallines Partikel nach einem der vorhergehenden Ansprüche, wobei die Mizellen jeweils 10 bis 1000 Copolymermoleküle umfassen.

8. Organisches kristallines Partikel nach einem der vorhergehenden Ansprüche, wobei die Mizellen vernetzbar sind.

9. Organisches kristallines Partikel nach einem der vorhergehenden Ansprüche, wobei das Partikel vollständig mit den Mizellen beschichtet ist.

10. Organisches kristallines Partikel nach einem der vorhergehenden Ansprüche, wobei die Kerne der Mizellen eine Chemikalie enthalten.

11. Organisches kristallines Partikel nach Anspruch 10, wobei es sich bei der in den Kernen der Mizellen enthaltenen Chemikalie um ein Lichtschutzmittel handelt.

12. Organisches kristallines Partikel nach Anspruch 10, wobei es sich bei der in den Kernen der Mizellen enthaltenen Chemikalie um einen biologischen Wirkstoff handelt.

13. Organisches kristallines Partikel nach Anspruch 10, wobei es sich bei der in den Kernen der Mizellen enthaltenen Chemikalie um ein Adjuvans handelt.

14. Zusammensetzung, umfassend eine Vielzahl von beschichteten organischen kristallinen Partikeln, wobei jedes dieser Partikel ein organisches kristallines Partikel nach einem der vorhergehenden Ansprüche ist.

15. Zusammensetzung nach Anspruch 14, wobei die Zusammensetzung eine feste Zusammensetzung ist.

16. Zusammensetzung nach Anspruch 14, wobei die organischen kristallinen Partikel in eine Flüssigkeit dispergiert sind.

17. Zusammensetzung nach Anspruch 16, wobei das Gewichtsverhältnis des Copolymers zu der Flüssigkeit von 1:100 000 zu 1:1 reicht.

18. Zusammensetzung nach Anspruch 16 oder 17, wobei die Flüssigkeit Wasser umfasst.

19. Zusammensetzung nach Anspruch 16 oder 17, wobei die Flüssigkeit nichtwässrig ist.

20. Zusammensetzung nach Anspruch 19, wobei die Mizellen reverse Mizellen sind.

21. Verfahren zur Herstellung eines beschichteten organischen kristallinen Partikels nach einem der Ansprüche 1 bis 13, umfassend die folgenden Schritte:
(a) Bilden von Mizellen des Copolymers; und
(b) Mischen der Mizellen mit einem organischen kristallinen Partikel.

22. Verfahren nach Anspruch 21, wobei die Mizellen vor dem Mischen, während des Mischens oder nach dem Mischen der Mizellen mit dem organischen kristallinen Partikel vernetzt werden.

23. Verwendung eines organischen kristallinen Partikels nach einem der Ansprüche 1 bis 13 oder Verwendung einer Zusammensetzung nach einem der Ansprüche 14 bis 20 zum Bekämpfen oder Kontrollieren eines landwirtschaftlichen Schädlings, wobei das organische kristalline Partikel ein biologisch aktiver Wirkstoff, bei dem es sich um eine Agrarchemikalie handelt, ist oder einen biologisch aktiven Wirkstoff, bei dem es sich um eine Agrarchemikalie handelt, umfasst.

## Revendications

1. Particule cristalline organique revêtue par au moins 10 micelles qui comprennent elles-mêmes un copolymère bloc AB qui comprend (i) un premier bloc hydrophobe A, comprenant un polymère choisi dans le groupe constitué par un homopolymère d'un monomère d'acrylate ou d'acrylate d'alkyle ; un copolymère comprenant deux ou trois monomères différents choisis parmi les monomères d'acrylate ou d'acrylate d'alkyle ; un homopolymère d'un monomère dérivé de styrène ; un copolymère comprenant deux monomères différents choisis parmi des monomères dérivés de styrène ; un homopolymère d'un monomère d'alcène ou de diène ; un copolymère comprenant deux monomères différents choisis parmi des monomères d'alcène ou de diène ; un homopolymère d'un monomère hétérocyclique ; et un copolymère statistique, alterné, à gradient ou bloc comprenant des monomères choisis parmi les monomères d'acrylate, les monomères d'acrylate d'alkyle, les monomères dérivés de styrène, les monomères d'alcène et les monomères de diène ; et (ii) un deuxième bloc B hydrophobe ou un bloc B hydrophile ayant une affinité différente par rapport au bloc A pour le milieu liquide dans lequel les copolymères AB sont dispersés, de façon à former des micelles.

2. Particule cristalline organique selon la revendication 1, dans laquelle la particule est ou comprend un composé biologiquement actif.

3. Particule cristalline organique selon la revendication 2, dans laquelle le composé biologiquement actif est une substance agrochimique ou pharmaceutique.

4. Particule cristalline organique selon l'une quelconque des revendications précédentes, dans laquelle la dimension la plus grande de la particule va de 5 mm à 10 nm.

5. Particule cristalline organique selon l'une quelconque des revendications précédentes, dans laquelle le polymère possède un poids moléculaire allant de 3000 à 100 000 Daltons.

6. Particule cristalline organique selon l'une quelconque des revendications précédentes, dans laquelle les micelles ont une dimension la plus grande allant de 3 à 500 nm.

7. Particule cristalline organique selon l'une quelconque des revendications précédentes, dans laquelle les micelles comprennent chacune de 10 à 1000 molécules de copolymère.

8. Particule cristalline organique selon l'une quelconque des revendications précédentes, dans laquelle les micelles sont réticulables.

9. Particule cristalline organique selon l'une quelconque des revendications précédentes, dans laquelle la particule est entièrement revêtue par les micelles.

10. Particule cristalline organique selon l'une quelconque des revendications précédentes, dans laquelle les coeurs des micelles contiennent une substance chimique.

11. Particule cristalline organique selon la revendication 10, dans laquelle la substance chimique contenue dans les coeurs des micelles est un photo-protecteur.

12. Particule cristalline organique selon la revendication 10, dans laquelle la substance chimique contenue dans les coeurs des micelles est un composé biologiquement actif.

13. Particule cristalline organique selon la revendication 10, dans laquelle la substance chimique contenue dans les coeurs des micelles est un adjuvant.

14. Composition comprenant une pluralité de particules cristallines organiques revêtues, dans laquelle chacune parmi ces particules est une particule cristalline organique selon l'une quelconque des revendications précédentes.

15. Composition selon la revendication 14, où la composition est une composition solide.

16. Composition selon la revendication 14, dans laquelle les particules cristallines organiques sont dispersées dans un liquide.

17. Composition selon la revendication 16, dans laquelle le rapport pondéral du copolymère au liquide va de 1:100 000 à 1:1.

18. Composition selon la revendication 16 ou 17, dans laquelle le liquide comprend de l'eau.

19. Composition selon la revendication 16 ou 17, dans laquelle le liquide est non aqueux.

20. Composition selon la revendication 19, dans laquelle les micelles sont des micelles inversées.

21. Procédé de préparation d'une particule cristalline organique revêtue selon l'une quelconque des revendications 1 à 13, comprenant les étapes consistant à
(a) former des micelles du copolymère ; et
(b) mélanger les micelles avec une particule cristalline organique.

22. Procédé selon la revendication 21, dans laquelle les micelles sont réticulées avant, pendant ou après le mélange des micelles avec la particule cristalline organique.

23. Utilisation d'une particule cristalline organique selon l'une quelconque des revendications 1 à 13 ou utilisation d'une composition selon l'une quelconque des revendications 14 à 20, pour lutter contre ou contrôler un nuisible agricole, dans laquelle la particule cristalline organique est ou comprend un composé biologiquement actif qui est une substance agrochimique.
